# EUROPEAN PATENT APPLICATION

(11) **EP 4 080 220 A1**
(43) Date of publication of application: **26.10.2022**
(21) Application number: 21169229.8
(22) Date of filing: 19.04.2021
(51) Int. Cl.: G01N 33/92

(54) **METHOD AND KIT FOR PREDICTING PREECLAMPSIA**

(71) Applicant: Max-Delbrück-Centrum für Molekulare Medizin in der Helmholtz-Gemeinschaft, 13125 Berlin (DE)
(72) Inventor: Herse, Florian, 13467 Berlin (DE); Bjorkholt Andersen, Louise, 2800 Kongens Lyngby (DK); Busjahn, Andreas, 16341 Panketal (DE); Dechend, Ralf, 16359 Biesenthal (DE)
(74) Representative: Hoppe, Georg Johannes

(57) **Abstract**

**Summary**

The invention pertains to a method for the prediction of preeclampsia in a pregnant female human subject until the end of gestational week 16 of pregnancy based on metabolites from a blood sample obtained from said subject. According to the invention, the method comprises the following steps:
- measuring the level of the following metabolites in the sample: 14,15-DHET, 13-HODE, 10,11-EDP, 21-HDHA, and 11,12-DHET, and
- concluding based on the measured level whether the subject is likely to develop preeclampsia.

SEQUENCE LISTING

<110>  Max-Delbrück-Centrum für Molekulare Medizin in der
       Helmholtz-Gemeinschaft

<120>  METHOD AND KIT FOR PREDICTING PREECLAMPSIA

<130>  MDC008EP

<160>  13

<170>  PatentIn version 3.5

<210>  1
<211>  1338
<212>  PRT
<213>  Homo sapiens

<400>  1

Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
            20                  25                  30

Glu Leu Ser Leu Lys Gly Thr Gln His Ile Met Gln Ala Gly Gln Thr
        35                  40                  45

Leu His Leu Gln Cys Arg Gly Glu Ala Ala His Lys Trp Ser Leu Pro
    50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr
                85                  90                  95

Ala Gln Ala Asn His Thr Gly Phe Tyr Ser Cys Lys Tyr Leu Ala Val
            100                 105                 110

Pro Thr Ser Lys Lys Lys Glu Thr Glu Ser Ala Ile Tyr Ile Phe Ile
        115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
        180                 185                 190

Ile Ile Ser Asn Ala Thr Tyr Lys Glu Ile Gly Leu Leu Thr Cys Glu
    195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240

Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys
            260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495

Asn Met Gly Asn Arg Ile Glu Ser Ile Thr Gln Arg Met Ala Ile Ile
            500                 505                 510

Glu Gly Lys Asn Lys Met Ala Ser Thr Leu Val Val Ala Asp Ser Arg
        515                 520                 525

Ile Ser Gly Ile Tyr Ile Cys Ile Ala Ser Asn Lys Val Gly Thr Val
    530                 535                 540

Gly Arg Asn Ile Ser Phe Tyr Ile Thr Asp Val Pro Asn Gly Phe His
545                 550                 555                 560

Val Asn Leu Glu Lys Met Pro Thr Glu Gly Glu Asp Leu Lys Leu Ser
                565                 570                 575

Cys Thr Val Asn Lys Phe Leu Tyr Arg Asp Val Thr Trp Ile Leu Leu
            580                 585                 590

Arg Thr Val Asn Asn Arg Thr Met His Tyr Ser Ile Ser Lys Gln Lys
        595                 600                 605

Met Ala Ile Thr Lys Glu His Ser Ile Thr Leu Asn Leu Thr Ile Met
    610                 615                 620

Asn Val Ser Leu Gln Asp Ser Gly Thr Tyr Ala Cys Arg Ala Arg Asn
625                 630                 635                 640

Val Tyr Thr Gly Glu Glu Ile Leu Gln Lys Lys Glu Ile Thr Ile Arg
                645                 650                 655

Asp Gln Glu Ala Pro Tyr Leu Leu Arg Asn Leu Ser Asp His Thr Val
            660                 665                 670

Ala Ile Ser Ser Ser Thr Thr Leu Asp Cys His Ala Asn Gly Val Pro

675                 680                 685

Glu Pro Gln Ile Thr Trp Phe Lys Asn Asn His Lys Ile Gln Gln Glu
    690                 695                 700

Pro Gly Ile Ile Leu Gly Pro Gly Ser Ser Thr Leu Phe Ile Glu Arg
705                 710                 715                 720

Val Thr Glu Glu Asp Glu Gly Val Tyr His Cys Lys Ala Thr Asn Gln
                725                 730                 735

Lys Gly Ser Val Glu Ser Ser Ala Tyr Leu Thr Val Gln Gly Thr Ser
            740                 745                 750

Asp Lys Ser Asn Leu Glu Leu Ile Thr Leu Thr Cys Thr Cys Val Ala
        755                 760                 765

Ala Thr Leu Phe Trp Leu Leu Leu Thr Leu Phe Ile Arg Lys Met Lys
    770                 775                 780

Arg Ser Ser Ser Glu Ile Lys Thr Asp Tyr Leu Ser Ile Ile Met Asp
785                 790                 795                 800

Pro Asp Glu Val Pro Leu Asp Glu Gln Cys Glu Arg Leu Pro Tyr Asp
                805                 810                 815

Ala Ser Lys Trp Glu Phe Ala Arg Glu Arg Leu Lys Leu Gly Lys Ser
            820                 825                 830

Leu Gly Arg Gly Ala Phe Gly Lys Val Val Gln Ala Ser Ala Phe Gly
        835                 840                 845

Ile Lys Lys Ser Pro Thr Cys Arg Thr Val Ala Val Lys Met Leu Lys
    850                 855                 860

Glu Gly Ala Thr Ala Ser Glu Tyr Lys Ala Leu Met Thr Glu Leu Lys
865                 870                 875                 880

Ile Leu Thr His Ile Gly His His Leu Asn Val Val Asn Leu Leu Gly
                885                 890                 895

Ala Cys Thr Lys Gln Gly Gly Pro Leu Met Val Ile Val Glu Tyr Cys
            900                 905                 910

Lys Tyr Gly Asn Leu Ser Asn Tyr Leu Lys Ser Lys Arg Asp Leu Phe
        915                 920                 925

Phe Leu Asn Lys Asp Ala Ala Leu His Met Glu Pro Lys Lys Glu Lys
    930                 935                 940

Met Glu Pro Gly Leu Glu Gln Gly Lys Lys Pro Arg Leu Asp Ser Val
945                 950                 955                 960

Thr Ser Ser Glu Ser Phe Ala Ser Ser Gly Phe Gln Glu Asp Lys Ser
                965                 970                 975

Leu Ser Asp Val Glu Glu Glu Glu Asp Ser Asp Gly Phe Tyr Lys Glu
            980                 985                 990

Pro Ile Thr Met Glu Asp Leu Ile Ser Tyr Ser Phe Gln Val Ala Arg
        995                 1000                1005

Gly Met Glu Phe Leu Ser Ser Arg Lys Cys Ile His Arg Asp Leu
    1010                1015                1020

Ala Ala Arg Asn Ile Leu Leu Ser Glu Asn Asn Val Val Lys Ile
    1025                1030                1035

Cys Asp Phe Gly Leu Ala Arg Asp Ile Tyr Lys Asn Pro Asp Tyr
    1040                1045                1050

Val Arg Lys Gly Asp Thr Arg Leu Pro Leu Lys Trp Met Ala Pro
    1055                1060                1065

Glu Ser Ile Phe Asp Lys Ile Tyr Ser Thr Lys Ser Asp Val Trp
    1070                1075                1080

Ser Tyr Gly Val Leu Leu Trp Glu Ile Phe Ser Leu Gly Gly Ser
    1085                1090                1095

Pro Tyr Pro Gly Val Gln Met Asp Glu Asp Phe Cys Ser Arg Leu
    1100                1105                1110

Arg Glu Gly Met Arg Met Arg Ala Pro Glu Tyr Ser Thr Pro Glu
    1115                1120                1125

Ile Tyr Gln Ile Met Leu Asp Cys Trp His Arg Asp Pro Lys Glu
    1130                1135                1140

Arg Pro Arg Phe Ala Glu Leu Val Glu Lys Leu Gly Asp Leu Leu
    1145                1150                1155

Gln Ala Asn Val Gln Gln Asp Gly Lys Asp Tyr Ile Pro Ile Asn
    1160                1165                1170

Ala Ile Leu Thr Gly Asn Ser Gly Phe Thr Tyr Ser Thr Pro Ala
    1175                1180                1185

Phe Ser Glu Asp Phe Phe Lys Glu Ser Ile Ser Ala Pro Lys Phe
    1190                1195                1200

Asn Ser Gly Ser Ser Asp Asp Val Arg Tyr Val Asn Ala Phe Lys
    1205                1210                1215

Phe Met Ser Leu Glu Arg Ile Lys Thr Phe Glu Glu Leu Leu Pro
    1220                1225                1230

Asn Ala Thr Ser Met Phe Asp Asp Tyr Gln Gly Asp Ser Ser Thr
    1235                1240                1245

Leu Leu Ala Ser Pro Met Leu Lys Arg Phe Thr Trp Thr Asp Ser
    1250                1255                1260

Lys Pro Lys Ala Ser Leu Lys Ile Asp Leu Arg Val Thr Ser Lys
    1265                1270                1275

Ser Lys Glu Ser Gly Leu Ser Asp Val Ser Arg Pro Ser Phe Cys
    1280                1285                1290

His Ser Ser Cys Gly His Val Ser Glu Gly Lys Arg Arg Phe Thr
    1295                1300                1305

Tyr Asp His Ala Glu Leu Glu Arg Lys Ile Ala Cys Cys Ser Pro
    1310                1315                1320

Pro Pro Asp Tyr Asn Ser Val Val Leu Tyr Ser Thr Pro Pro Ile
    1325                1330                1335

<210> 2
<211> 687
<212> PRT
<213> Homo sapiens

<400> 2

Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
            20                  25                  30

Glu Leu Ser Leu Lys Gly Thr Gln His Ile Met Gln Ala Gly Gln Thr
        35                  40                  45

Leu His Leu Gln Cys Arg Gly Glu Ala Ala His Lys Trp Ser Leu Pro
    50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr
                85                  90                  95

Ala Gln Ala Asn His Thr Gly Phe Tyr Ser Cys Lys Tyr Leu Ala Val
            100                 105                 110

Pro Thr Ser Lys Lys Lys Glu Thr Glu Ser Ala Ile Tyr Ile Phe Ile
        115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
            180                 185                 190

Ile Ile Ser Asn Ala Thr Tyr Lys Glu Ile Gly Leu Leu Thr Cys Glu
        195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240

Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys
            260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495

Asn Met Gly Asn Arg Ile Glu Ser Ile Thr Gln Arg Met Ala Ile Ile
            500                 505                 510

Glu Gly Lys Asn Lys Met Ala Ser Thr Leu Val Val Ala Asp Ser Arg
        515                 520                 525

Ile Ser Gly Ile Tyr Ile Cys Ile Ala Ser Asn Lys Val Gly Thr Val
    530                 535                 540

Gly Arg Asn Ile Ser Phe Tyr Ile Thr Asp Val Pro Asn Gly Phe His

545                 550                 555                 560

Val Asn Leu Glu Lys Met Pro Thr Glu Gly Glu Asp Leu Lys Leu Ser
                565                 570                 575

Cys Thr Val Asn Lys Phe Leu Tyr Arg Asp Val Thr Trp Ile Leu Leu
            580                 585                 590

Arg Thr Val Asn Asn Arg Thr Met His Tyr Ser Ile Ser Lys Gln Lys
        595                 600                 605

Met Ala Ile Thr Lys Glu His Ser Ile Thr Leu Asn Leu Thr Ile Met
    610                 615                 620

Asn Val Ser Leu Gln Asp Ser Gly Thr Tyr Ala Cys Arg Ala Arg Asn
625                 630                 635                 640

Val Tyr Thr Gly Glu Glu Ile Leu Gln Lys Lys Glu Ile Thr Ile Arg
                645                 650                 655

Gly Glu His Cys Asn Lys Lys Ala Val Phe Ser Arg Ile Ser Lys Phe
            660                 665                 670

Lys Ser Thr Arg Asn Asp Cys Thr Thr Gln Ser Asn Val Lys His
        675                 680                 685

<210>  3
<211>  733
<212>  PRT
<213>  Homo sapiens

<400>  3

Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
            20                  25                  30

Glu Leu Ser Leu Lys Gly Thr Gln His Ile Met Gln Ala Gly Gln Thr
        35                  40                  45

Leu His Leu Gln Cys Arg Gly Glu Ala Ala His Lys Trp Ser Leu Pro
    50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr

                85                  90                  95

Ala Gln Ala Asn His Thr Gly Phe Tyr Ser Cys Lys Tyr Leu Ala Val
            100                 105                 110

Pro Thr Ser Lys Lys Lys Glu Thr Glu Ser Ala Ile Tyr Ile Phe Ile
        115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
            180                 185                 190

Ile Ile Ser Asn Ala Thr Tyr Lys Glu Ile Gly Leu Leu Thr Cys Glu
        195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240

Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys
            260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495

Asn Met Gly Asn Arg Ile Glu Ser Ile Thr Gln Arg Met Ala Ile Ile
            500                 505                 510

Glu Gly Lys Asn Lys Met Ala Ser Thr Leu Val Val Ala Asp Ser Arg
        515                 520                 525

Ile Ser Gly Ile Tyr Ile Cys Ile Ala Ser Asn Lys Val Gly Thr Val
    530                 535                 540

Gly Arg Asn Ile Ser Phe Tyr Ile Thr Asp Val Pro Asn Gly Phe His
545                 550                 555                 560

Val Asn Leu Glu Lys Met Pro Thr Glu Gly Glu Asp Leu Lys Leu Ser
                565                 570                 575

Cys Thr Val Asn Lys Phe Leu Tyr Arg Asp Val Thr Trp Ile Leu Leu
            580                 585                 590

Arg Thr Val Asn Asn Arg Thr Met His Tyr Ser Ile Ser Lys Gln Lys
        595                 600                 605

Met Ala Ile Thr Lys Glu His Ser Ile Thr Leu Asn Leu Thr Ile Met
    610                 615                 620

Asn Val Ser Leu Gln Asp Ser Gly Thr Tyr Ala Cys Arg Ala Arg Asn
625                 630                 635                 640

Val Tyr Thr Gly Glu Glu Ile Leu Gln Lys Lys Glu Ile Thr Ile Arg
                645                 650                 655

Asp Gln Glu Ala Pro Tyr Leu Leu Arg Asn Leu Ser Asp His Thr Val
            660                 665                 670

Ala Ile Ser Ser Ser Thr Thr Leu Asp Cys His Ala Asn Gly Val Pro
        675                 680                 685

Glu Pro Gln Ile Thr Trp Phe Lys Asn Asn His Lys Ile Gln Gln Glu
    690                 695                 700

Pro Glu Leu Tyr Thr Ser Thr Ser Pro Ser Ser Ser Ser Ser Ser Pro
705                 710                 715                 720

Leu Ser Ser Ser Ser Ser Ser Ser Ser Ser Ser Ser Ser
                725                 730

<210>  4
<211>  541
<212>  PRT
<213>  Homo sapiens

<400>  4

Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
            20                  25                  30

Glu Leu Ser Leu Lys Gly Thr Gln His Ile Met Gln Ala Gly Gln Thr
        35                  40                  45

Leu His Leu Gln Cys Arg Gly Glu Ala Ala His Lys Trp Ser Leu Pro
    50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr
                85                  90                  95

Ala Gln Ala Asn His Thr Gly Phe Tyr Ser Cys Lys Tyr Leu Ala Val
            100                 105                 110

Pro Thr Ser Lys Lys Lys Glu Thr Glu Ser Ala Ile Tyr Ile Phe Ile
        115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
            180                 185                 190

Ile Ile Ser Asn Ala Thr Tyr Lys Glu Ile Gly Leu Leu Thr Cys Glu
        195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240

Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys
            260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495

Asn Met Gly Asn Arg Ile Glu Ser Ile Thr Gln Arg Met Ala Ile Ile
            500                 505                 510

Glu Gly Lys Asn Lys Leu Pro Pro Ala Asn Ser Ser Phe Met Leu Pro
        515                 520                 525

Pro Thr Ser Phe Ser Ser Asn Tyr Phe His Phe Leu Pro
    530                 535                 540

<210>  5
<211>  665
<212>  PRT
<213>  Homo sapiens

<400>  5

Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
                20                  25                  30

Glu Leu Ser Leu Lys Gly Thr Gln His Ile Met Gln Ala Gly Gln Thr
            35                  40                  45

Leu His Leu Gln Cys Arg Gly Glu Ala Ala His Lys Trp Ser Leu Pro
    50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr
                85                  90                  95

Ala Gln Ala Asn His Thr Gly Phe Tyr Ser Cys Lys Tyr Leu Ala Val
                100                 105                 110

Pro Thr Ser Lys Lys Lys Glu Thr Glu Ser Ala Ile Tyr Ile Phe Ile
            115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
                180                 185                 190

Ile Ile Ser Asn Ala Thr Tyr Lys Glu Ile Gly Leu Leu Thr Cys Glu
            195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240

Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys

260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495

Asn Met Gly Asn Arg Ile Glu Ser Ile Thr Gln Arg Met Ala Ile Ile
            500                 505                 510

Glu Gly Lys Asn Lys Met Ala Ser Thr Leu Val Val Ala Asp Ser Arg
        515                 520                 525

Ile Ser Gly Ile Tyr Ile Cys Ile Ala Ser Asn Lys Val Gly Thr Val
    530                 535                 540

Gly Arg Asn Ile Ser Phe Tyr Ile Thr Asp Val Pro Asn Gly Phe His
545                 550                 555                 560

Val Asn Leu Glu Lys Met Pro Thr Glu Gly Glu Asp Leu Lys Leu Ser
                565                 570                 575

Cys Thr Val Asn Lys Phe Leu Tyr Arg Asp Val Thr Trp Ile Leu Leu
            580                 585                 590

Arg Thr Val Asn Asn Arg Thr Met His Tyr Ser Ile Ser Lys Gln Lys
        595                 600                 605

Met Ala Ile Thr Lys Glu His Ser Ile Thr Leu Asn Leu Thr Ile Met
    610                 615                 620

Asn Val Ser Leu Gln Asp Ser Gly Thr Tyr Ala Cys Arg Ala Arg Asn
625                 630                 635                 640

Val Tyr Thr Gly Glu Glu Ile Leu Gln Lys Lys Glu Ile Thr Ile Arg
                645                 650                 655

Gly Ser Glu Glu Met Leu Leu Pro Lys
            660                 665

<210>  6
<211>  85
<212>  PRT
<213>  Homo sapines

<400>  6

Met Leu Lys Arg Phe Thr Trp Thr Asp Ser Lys Pro Lys Ala Ser Leu
1               5                   10                  15

Lys Ile Asp Leu Arg Val Thr Ser Lys Ser Lys Glu Ser Gly Leu Ser
            20                  25                  30

Asp Val Ser Arg Pro Ser Phe Cys His Ser Ser Cys Gly His Val Ser
        35                  40                  45

Glu Gly Lys Arg Arg Phe Thr Tyr Asp His Ala Glu Leu Glu Arg Lys
    50                  55                  60

Ile Ala Cys Cys Ser Pro Pro Pro Asp Tyr Asn Ser Val Val Leu Tyr
65                  70                  75                  80

Ser Thr Pro Pro Ile
                85

<210>  7
<211>  433
<212>  PRT
<213>  Homo sapiens

<400>  7

Met Val Ile Val Glu Tyr Cys Lys Tyr Gly Asn Leu Ser Asn Tyr Leu
1               5                   10                  15

Lys Ser Lys Arg Asp Leu Phe Phe Leu Asn Lys Asp Ala Ala Leu His
            20                  25                  30

Met Glu Pro Lys Lys Glu Lys Met Glu Pro Gly Leu Glu Gln Gly Lys
        35                  40                  45

Lys Pro Arg Leu Asp Ser Val Thr Ser Ser Glu Ser Phe Ala Ser Ser
    50                  55                  60

Gly Phe Gln Glu Asp Lys Ser Leu Ser Asp Val Glu Glu Glu Glu Asp
65                  70                  75                  80

Ser Asp Gly Phe Tyr Lys Glu Pro Ile Thr Met Glu Asp Leu Ile Ser
                85                  90                  95

Tyr Ser Phe Gln Val Ala Arg Gly Met Glu Phe Leu Ser Ser Arg Lys
            100                 105                 110

Cys Ile His Arg Asp Leu Ala Ala Arg Asn Ile Leu Leu Ser Glu Asn
        115                 120                 125

Asn Val Val Lys Ile Cys Asp Phe Gly Leu Ala Arg Asp Ile Tyr Lys
    130                 135                 140

Asn Pro Asp Tyr Val Arg Lys Gly Asp Thr Arg Leu Pro Leu Lys Trp
145                 150                 155                 160

Met Ala Pro Glu Ser Ile Phe Asp Lys Ile Tyr Ser Thr Lys Ser Asp
                165                 170                 175

Val Trp Ser Tyr Gly Val Leu Leu Trp Glu Ile Phe Ser Leu Gly Gly
            180                 185                 190

Ser Pro Tyr Pro Gly Val Gln Met Asp Glu Asp Phe Cys Ser Arg Leu
        195                 200                 205

Arg Glu Gly Met Arg Met Arg Ala Pro Glu Tyr Ser Thr Pro Glu Ile
    210                 215                 220

Tyr Gln Ile Met Leu Asp Cys Trp His Arg Asp Pro Lys Glu Arg Pro
225                 230                 235                 240

Arg Phe Ala Glu Leu Val Glu Lys Leu Gly Asp Leu Leu Gln Ala Asn
                245                 250                 255

Val Gln Gln Asp Gly Lys Asp Tyr Ile Pro Ile Asn Ala Ile Leu Thr
            260                 265                 270

Gly Asn Ser Gly Phe Thr Tyr Ser Thr Pro Ala Phe Ser Glu Asp Phe
        275                 280                 285

Phe Lys Glu Ser Ile Ser Ala Pro Lys Phe Asn Ser Gly Ser Ser Asp
    290                 295                 300

Asp Val Arg Tyr Val Asn Ala Phe Lys Phe Met Ser Leu Glu Arg Ile
305                 310                 315                 320

Lys Thr Phe Glu Glu Leu Leu Pro Asn Ala Thr Ser Met Phe Asp Asp
                325                 330                 335

Tyr Gln Gly Asp Ser Ser Thr Leu Leu Ala Ser Pro Met Leu Lys Arg
            340                 345                 350

Phe Thr Trp Thr Asp Ser Lys Pro Lys Ala Ser Leu Lys Ile Asp Leu
        355                 360                 365

Arg Val Thr Ser Lys Ser Lys Glu Ser Gly Leu Ser Asp Val Ser Arg
    370                 375                 380

Pro Ser Phe Cys His Ser Ser Cys Gly His Val Ser Glu Gly Lys Arg
385                 390                 395                 400

Arg Phe Thr Tyr Asp His Ala Glu Leu Glu Arg Lys Ile Ala Cys Cys
                405                 410                 415

Ser Pro Pro Pro Asp Tyr Asn Ser Val Val Leu Tyr Ser Thr Pro Pro
            420                 425                 430

Ile

<210>  8
<211>  361
<212>  PRT
<213>  Homo sapiens

<400>  8

Met Asn Ser Asp Leu Leu Val Asp Ser Asp Gly Phe Tyr Lys Glu Pro
1               5                   10                  15

Ile Thr Met Glu Asp Leu Ile Ser Tyr Ser Phe Gln Val Ala Arg Gly
            20                  25                  30

Met Glu Phe Leu Ser Ser Arg Lys Cys Ile His Arg Asp Leu Ala Ala
        35                  40                  45

Arg Asn Ile Leu Leu Ser Glu Asn Asn Val Val Lys Ile Cys Asp Phe
    50                  55                  60

Gly Leu Ala Arg Asp Ile Tyr Lys Asn Pro Asp Tyr Val Arg Lys Gly
65                  70                  75                  80

Asp Thr Arg Leu Pro Leu Lys Trp Met Ala Pro Glu Ser Ile Phe Asp
                85                  90                  95

Lys Ile Tyr Ser Thr Lys Ser Asp Val Trp Ser Tyr Gly Val Leu Leu
            100                 105                 110

Trp Glu Ile Phe Ser Leu Gly Gly Ser Pro Tyr Pro Gly Val Gln Met
        115                 120                 125

Asp Glu Asp Phe Cys Ser Arg Leu Arg Glu Gly Met Arg Met Arg Ala
    130                 135                 140

Pro Glu Tyr Ser Thr Pro Glu Ile Tyr Gln Ile Met Leu Asp Cys Trp
145                 150                 155                 160

His Arg Asp Pro Lys Glu Arg Pro Arg Phe Ala Glu Leu Val Glu Lys
                165                 170                 175

Leu Gly Asp Leu Leu Gln Ala Asn Val Gln Gln Asp Gly Lys Asp Tyr
            180                 185                 190

Ile Pro Ile Asn Ala Ile Leu Thr Gly Asn Ser Gly Phe Thr Tyr Ser
        195                 200                 205

Thr Pro Ala Phe Ser Glu Asp Phe Phe Lys Glu Ser Ile Ser Ala Pro
    210                 215                 220

Lys Phe Asn Ser Gly Ser Ser Asp Asp Val Arg Tyr Val Asn Ala Phe
225                 230                 235                 240

Lys Phe Met Ser Leu Glu Arg Ile Lys Thr Phe Glu Glu Leu Leu Pro
                245                 250                 255

Asn Ala Thr Ser Met Phe Asp Asp Tyr Gln Gly Asp Ser Ser Thr Leu
            260                 265                 270

Leu Ala Ser Pro Met Leu Lys Arg Phe Thr Trp Thr Asp Ser Lys Pro
        275                 280                 285

Lys Ala Ser Leu Lys Ile Asp Leu Arg Val Thr Ser Lys Ser Lys Glu
    290                 295                 300

Ser Gly Leu Ser Asp Val Ser Arg Pro Ser Phe Cys His Ser Ser Cys
305                 310                 315                 320

Gly His Val Ser Glu Gly Lys Arg Arg Phe Thr Tyr Asp His Ala Glu
                325                 330                 335

Leu Glu Arg Lys Ile Ala Cys Cys Ser Pro Pro Pro Asp Tyr Asn Ser
            340                 345                 350

Val Val Leu Tyr Ser Thr Pro Pro Ile
        355                 360

<210>  9
<211>  85
<212>  PRT
<213>  Homo sapiens

<400>  9

Met Leu Lys Arg Phe Thr Trp Thr Asp Ser Lys Pro Lys Ala Ser Leu
1               5                   10                  15

Lys Ile Asp Leu Arg Val Thr Ser Lys Ser Lys Glu Ser Gly Leu Ser
            20                  25                  30

Asp Val Ser Arg Pro Ser Phe Cys His Ser Ser Cys Gly His Val Ser
        35                  40                  45

Glu Gly Lys Arg Arg Phe Thr Tyr Asp His Ala Glu Leu Glu Arg Lys
    50                  55                  60

Ile Ala Cys Cys Ser Pro Pro Pro Asp Tyr Asn Ser Val Val Leu Tyr
65                  70                  75                  80

Ser Thr Pro Pro Ile
                85

<210>  10
<211>  169
<212>  PRT
<213>  Homo sapiens

<400>  10

Met Pro Val Met Arg Leu Phe Pro Cys Phe Leu Gln Leu Leu Ala Gly
1               5                   10                  15

Leu Ala Leu Pro Ala Val Pro Pro Gln Trp Ala Leu Ser Ala Gly Asn
            20                  25                  30

Gly Ser Ser Glu Val Glu Val Val Pro Phe Gln Glu Val Trp Gly Arg
        35                  40                  45

Ser Tyr Cys Arg Ala Leu Glu Arg Leu Val Asp Val Val Ser Glu Tyr
    50                  55                  60

Pro Ser Glu Val Glu His Met Phe Ser Pro Ser Cys Val Ser Leu Leu
65                  70                  75                  80

Arg Cys Thr Gly Cys Cys Gly Asp Glu Asn Leu His Cys Val Pro Val
                85                  90                  95

Glu Thr Ala Asn Val Thr Met Gln Leu Leu Lys Ile Arg Ser Gly Asp
            100                 105                 110

Arg Pro Ser Tyr Val Glu Leu Thr Phe Ser Gln His Val Arg Cys Glu
        115                 120                 125

Cys Arg Pro Leu Arg Glu Lys Met Lys Pro Glu Arg Arg Arg Pro Lys
    130                 135                 140

Gly Arg Gly Lys Arg Arg Arg Glu Lys Gln Arg Pro Thr Asp Cys His
145                 150                 155                 160

Leu Cys Gly Asp Ala Val Pro Arg Arg
                165

<210>  11
<211>  221
<212>  PRT
<213>  Homo sapiens

<400>  11

Met Pro Val Met Arg Leu Phe Pro Cys Phe Leu Gln Leu Leu Ala Gly
1               5                   10                  15

Leu Ala Leu Pro Ala Val Pro Pro Gln Gln Trp Ala Leu Ser Ala Gly
            20                  25                  30

Asn Gly Ser Ser Glu Val Glu Val Val Pro Phe Gln Glu Val Trp Gly
        35                  40                  45

Arg Ser Tyr Cys Arg Ala Leu Glu Arg Leu Val Asp Val Val Ser Glu
    50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95

Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg His Ser Pro Gly Arg Gln Ser Pro Asp Met Pro Gly Asp
    130                 135                 140

Phe Arg Ala Asp Ala Pro Ser Phe Leu Pro Pro Arg Arg Ser Leu Pro
145                 150                 155                 160

Met Leu Phe Arg Met Glu Trp Gly Cys Ala Leu Thr Gly Ser Gln Ser
                165                 170                 175

Ala Val Trp Pro Ser Ser Pro Val Pro Glu Glu Ile Pro Arg Met His
            180                 185                 190

Pro Gly Arg Asn Gly Lys Lys Gln Gln Arg Lys Pro Leu Arg Glu Lys
        195                 200                 205

Met Lys Pro Glu Arg Cys Gly Asp Ala Val Pro Arg Arg
    210                 215                 220

<210>  12
<211>  149
<212>  PRT
<213>  Homo sapiens

<400>  12

Met Pro Val Met Arg Leu Phe Pro Cys Phe Leu Gln Leu Leu Ala Gly
1               5                   10                  15

Leu Ala Leu Pro Ala Val Pro Pro Gln Gln Trp Ala Leu Ser Ala Gly
            20                  25                  30

Asn Gly Ser Ser Glu Val Glu Val Val Pro Phe Gln Glu Val Trp Gly
        35                  40                  45

Arg Ser Tyr Cys Arg Ala Leu Glu Arg Leu Val Asp Val Val Ser Glu
    50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95

Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg Pro Leu Arg Glu Lys Met Lys Pro Glu Arg Cys Gly Asp
    130                 135                 140

Ala Val Pro Arg Arg
145

<210>  13
<211>  170
<212>  PRT
<213>  Homo sapiens

<400>  13

Met Pro Val Met Arg Leu Phe Pro Cys Phe Leu Gln Leu Leu Ala Gly
1               5                   10                  15

Leu Ala Leu Pro Ala Val Pro Pro Gln Gln Trp Ala Leu Ser Ala Gly
            20                  25                  30

Asn Gly Ser Ser Glu Val Glu Val Val Pro Phe Gln Glu Val Trp Gly
        35                  40                  45

Arg Ser Tyr Cys Arg Ala Leu Glu Arg Leu Val Asp Val Val Ser Glu
    50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95

Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg Pro Leu Arg Glu Lys Met Lys Pro Glu Arg Arg Arg Pro
    130                 135                 140

Lys Gly Arg Gly Lys Arg Arg Arg Glu Lys Gln Arg Pro Thr Asp Cys
145                 150                 155                 160

His Leu Cys Gly Asp Ala Val Pro Arg Arg
                165                 170

## Description

The invention relates to a method for the prediction of preeclampsia in a pregnant female human subject until the end of gestational week 16 of pregnancy based on metabolites (markers) from a blood sample obtained from said subject.

### Description

Preeclampsia is a major cause of morbidity and mortality during pregnancy.

The preeclampsia syndrome features new-onset hypertension (>140/90 mm Hg) after gestational week 20 accompanied by proteinuria (>300 mg/24 hours).

Overall, 5 to 10% of all pregnancies worldwide develop preeclampsia, a leading cause of morbidity and mortality in mother and child.

The object of the present invention is to provide a means for predicting the likelihood of a pregnant women to suffer from preeclampsia.

The present invention provides methods and kits for predicting and screening for preeclampsia. Particularly, the invention provides for preparing metabolite profiles of subject's blood samples, the metabolite profiles being indicative of the presence or absence of the development of preeclampsia during pregnancy. The invention further provides for evaluating the subject metabolite profiles for the presence or absence of one or more metabolite signatures that are indicative of preeclampsia.

The inventors have surprisingly found that certain lipids, namely eicosanoids, signaling molecules derived from polyunsaturated fatty acids which exert complex control over several communication pathways in the human body, can be used to predict preeclampsia. They found that measuring the level of certain metabolites in the blood sample allows for the prediction of the presence or absence of preeclampsia with high predictive values.

Here, the term "eicosanoids" is used to include oxylipins that are derived from arachidonic acids (AA), linoleic acid (LA), or eicosapentaenoic- and docosahexaenoic-acid (EPA/DHA).

In other words, measuring these metabolite is necessary and sufficient for the prediction whether a pregnant female human subject will develop preeclampsia or not based on a blood sample obtained from said subject by measuring the level of metabolites in the sample, that are chosen from the metabolites listed herein, in particular from the metabolites in table 1, and concluding based on the measured levels whether the subject will develop preeclampsia or not.

In one aspect, the invention provides a method for preparing metabolite profiles that are indicative of the presence or absence of preeclampsia. The metabolite profiles are prepared from subject blood samples. The number of metabolites in the metabolite profile may be selected so as to offer a convenient and cost effective means for screening samples for the future presence or absence of preeclampsia with high sensitivity and high specificity. Generally, the metabolite profile includes the amount or the concentration (referred to here as "level") of from 5 to about 21 metabolites or more. The concentration can be inferred from the measured amount since the volume used in the method is known.

In a second aspect, the invention provides a method for predicting preeclampsia. In this aspect the method comprises preparing a metabolite profile by measuring the level of at least 5 metabolites in a subject's blood sample, where the level of such metabolites are indicative of the presence or absence of preeclampsia. The metabolites may be the ones listed in table 1, and exemplary sets of such profiles are disclosed herein, for example, in the figures. The method further comprises evaluating the profile for the presence or absence of a metabolite signature indicative of preeclampsia, to thereby conclude whether the subject will or will not develop preeclampsia.

Specifically, the method of the invention comprises at least the following steps: measuring the abundance of at least 5 metabolites in the sample that are chosen from the metabolites listed in table 1, and concluding, based on the measured levels, whether the subject has preeclampsia or not. Measuring the level of metabolites may comprise preparing serum or plasma from blood samples, and measuring levels of the metabolites using mass spectrometry. Alternatively, other methods for determining metabolite levels may be employed.

More specifically, the invention refers in one aspect to a method for the prediction of preeclampsia in a pregnant female human subject until the end of gestational week 16, preferably within the first trimester of pregnancy based on metabolites from a blood sample obtained from said subject. The method allows for a classification of subjects into healthy women and peeclamptic women.

The term "until the end of gestational week 16" refers to the time of pregnancy from gestational week 1 to the end of gestational week 16 of pregnancy. In certain embodiments, the method is used in the first trimester of pregnancy, i.e. from gestational week 1 to the end of gestational week 12 of pregnancy. The method may also be used from gestational week 4, 5, or 6 to gestational week 12 or 16.

In one embodiment, the method comprises the following steps:
- measuring the level of each of the following metabolites in the sample: 14,15-DHET, 13-HODE, 10,11-EDP, 21-HDHA, and 11,12-DHET, and
- concluding based on the measured level whether the subject is likely to develop preeclampsia.

The metabolites 14,15-DHET, 13-HODE, 10,11-EDP, 21-HDHA, and 11,12-DHET are listed in table 1.

The identities and/or combinations of metabolites that make up or are included in expression profiles are disclosed in table 1. In particular embodiments, measuring the level of metabolites listed in table 2 and/or table 3 may be included in the method.

**Table 1: Group of 5 Metabolite Markers**

| **Abbreviation** | **Chemical Name** |
|---|---|
| 14, 15-DHET | 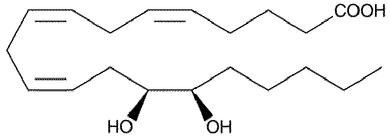 |
| | (±)14,15-dihydroxy-5Z,8Z,11Z-eicosatrienoic acid |
| 11-HETE | 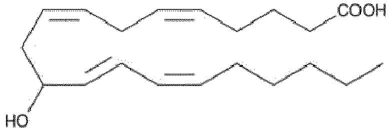 |
| | (±)11-HETE |
| | CAS: 73804-65-6 |
| 10,11-EDP | 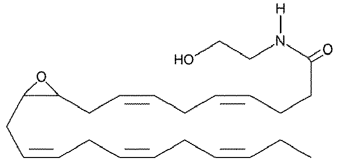 |
| | N-(2-hydroxyethyl)-9-[3-(2Z,5Z,8Z)-2,5,8-undecatrien-1-yl-2-oxiranyl]-4Z,7Z-nonadienamide |
| | CAS: 2123484-71-7 |
| 13-HODE | 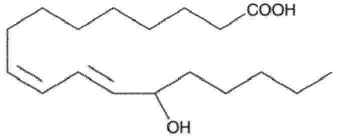 |
| | (±)-13-hydroxy-9Z,11E-octadecadienoic acid, |
| | CAS: 73804-64-5 |
| 21-HDHA | 21-hydroxy-4Z,7Z,10Z, 13Z, 16Z, 19Z-docosahexaenoic acid |

In certain embodiments of the method of the invention, the levels of additional markers to the five markers of table 1 are measured.

In certain embodiments of the method, the levels of at least one (1, 2, 3, or more) of the following metabolites are measured in the sample in addition to the metabolites of table 1: 7,8-EDP, 16,17-DiHDPA, 12,13-DiHOME, 11,12-DHET, 9,10-DiHOME, 5,6-DiHETE; 10-HDHA, 11,12-EET, 15-HETE, 7,8-DiHDPA, PGE2, 14,15-EET, 18-HETE, EODAcis, EODAtrans, and 13,14-DiHDPA. These markers are listed in table 2.

**Table 2: Group of additional 16 Metabolite Markers**

| **Abbreviation** | **Chemical/Alternative Name** |
|---|---|
| 7,8-EDP | 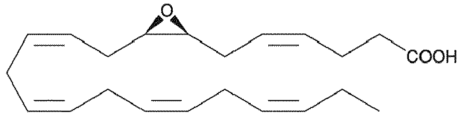 |
| | (±)-(4Z)-6-[3-(2Z,5Z,8Z,11Z)-2,5,8,11-tetradecatetraen-1-yl-2-oxiranyl]-4-hexenoic acid |
| | CAS: 895127-66-9 |
| 16,17-DiHDPA | 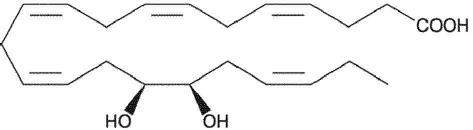 |
| | (±) 16,17 -dihydroxy-4Z, 7Z, 10Z, 13Z, 19Z-docosapentaenoic acid |
| | CAS: 1345275-27-5 |
| 12,13-DiHOME | 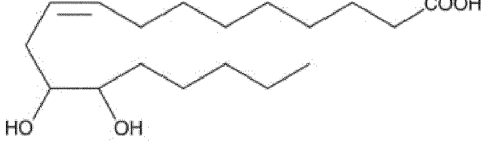 |
| | 12,13-dihydroxy-9Z-octadecenoic acid |
| | CAS: 263399-35-5 |
| 11,12-DHET | 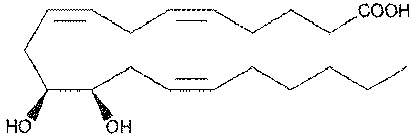 |
| | (±)11,12-dihydroxy-5Z,8Z,14Z-eicosatrienoic acid |
| 9,10-DiHOME | 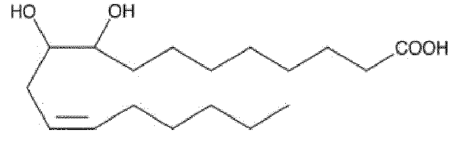 |
| | CAS: 263399-34-4 |
| 5,6-DiHETE | 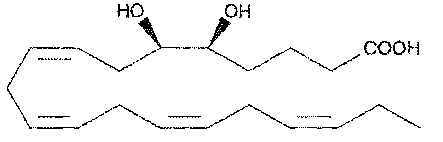 |
| | (±)5,6-dihydroxy-8Z,11Z,14Z,17Z-eicosatetraenoic acid |
| | CAS: 845673-97-4 |
| 10-HDHA | 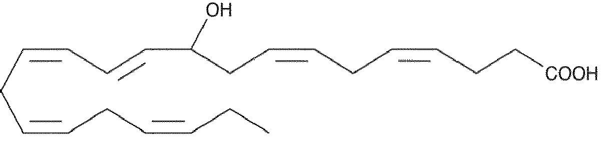 |
| | (±)10-hydroxy-4Z,7Z,11E,13Z,16Z,19Z-docosahexaenoic acid |
| | CAS: 90780-50-0 |
| 11,12-EET | 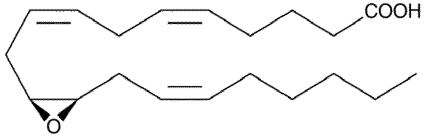 |
| | (±)11(12)-epoxy- 5Z, 8Z, 14Z- eicosatrienoic acid |
| | CAS: 81276-02-0 |
| 15-HETE | 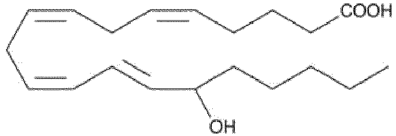 |
| | CAS: 73836-87-0 |
| 7,8-DiHDPA | 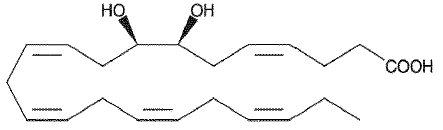 |
| | (±)7,8-dihydroxydocosa-4Z,10Z,13Z, 16Z,19Z-pentaenoic acid |
| | CAS: 168111-93-1 |
| PGE2 | 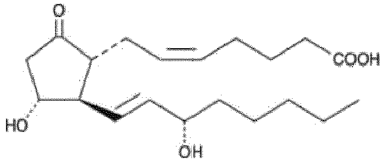 |
| | Prostaglandin E2 |
| | CAS: 363-24-6 |
| 14,15-EET | 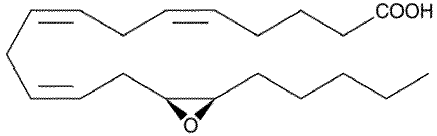 |
| | (±)14,15-DiHETrE |
| 18-HETE | 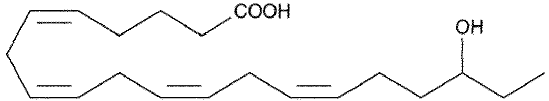 |
| | (±)18-hydroxy-5Z,8Z,11Z,14Z-eicosatetraenoic acid |
| | CAS: 133268-58-3 |
| EODAcis | 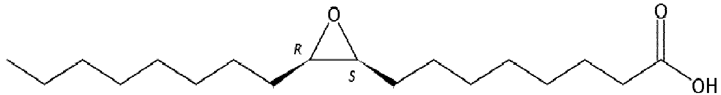 |
| | 9,10-epoxyoctadecanoic acid; (±)-cis-9,10-epoxyoctadecanoic acid |
| | CAS: 24560-98-3 |
| EODAtrans | 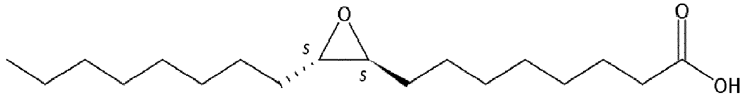 |
| | trans-9,10-epoxystearic acid; (±)-trans-9,10-epoxyoctadecanoic acid |
| | CAS: 13980-07-09 |
| 13,14-DiHDPA | 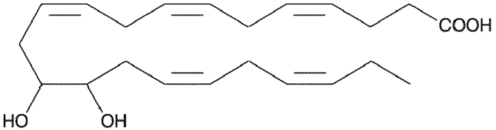 |
| | 13, 14-dihydroxy-4Z, 7Z, 10Z, 16Z, 19Z-docosapentaenoic acid |
| | CAS: 1345275-24-2 |

In preferred embodiments of the method, the levels of five or more (6, 7, 8, ... 16) of the metabolites of table 2 are measured in the sample in addition to the markers listed in table 1.

In certain embodiments, a metabolite listed in table 3 is measured in addition to the metabolites listed in table 1 and, optionally, one or more of the metabolites listed in table 2.

**Table 3: Group of additional Metabolite Markers**

| **Abbreviation** | **Chemical/Alternative Name** |
|---|---|
| 11-HETE | 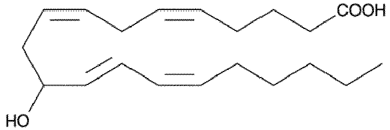 |
| | ((±)11-hydroxy-5Z,8Z,12E,14Z-eicosatetraenoic acid |
| | CAS: 73804-65-6 |
| 15-keto-PGE2 | 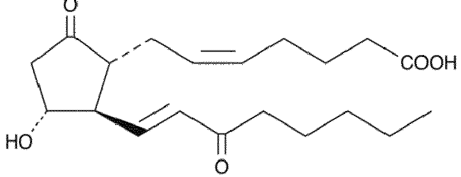 |
| | 9,15-dioxo-11α-hydroxy-prosta-5Z,13E-dien-1-oic acid |
| | CAS: 26441-05-4 |
| 13,14-dihydro-15-keto-PGE2 | 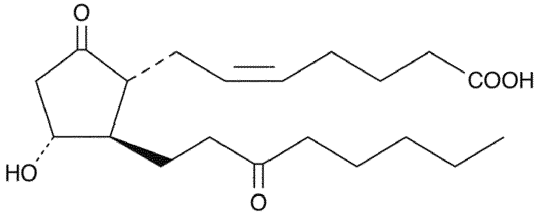 |
| | 9,15-dioxo-11α-hydroxy-prost-5Z-en-1-oic acid |
| | CAS : 363-23-5 |

In certain embodiments of the method of the invention, the of level of 11-HETE shown in table 3 is additionally measured in the sample in addition to the metabolites listed in table 2 and possibly other metabolites, such as other metabolites listed in table 3.

In certain embodiments of the method of the invention, additional metabolites are measured. Such additional metabolites may be any eicosanoid.

Preferred embodiments of the method of the invention are disclosed throughout the application, in particular in the figures.

In one preferred embodiment of the invention, the method comprises the following steps:
- measuring the level of the metabolites listed in table 1 in the sample (14,15-DHET, 13-HODE, 10,11-EDP, 21-HDHA, and 11-HETE) together with the following selection of markers of table 2: 5,6-DiHETE; 11,12-DHET; 13,14-DiHDPA; 12,13-DiHOME; 9,10-DiHOME; 18-HETE, and
- concluding based on the measured level whether the subject is likely to develop preeclampsia.

In another preferred embodiment, the method comprises the following steps:
- measuring the level of the metabolites listed in table 1 in the sample (14,15-DHET, 13-HODE, 10,11-EDP, 21-HDHA, and 11-HETE) together with the following selection of markers of table 2: 5,6-DiHETE; 11,12-DHET; 13,14-DiHDPA; 12,13-DiHOME; 9,10-DiHOME; 18-HETE; 14,15-EET, and
- concluding based on the measured level whether the subject is likely to develop preeclampsia.

In certain embodiments, the level of the angiogenic marker concentrations soluble *fms*-like tyrosine kinase 1 (sFlt-1) (SEQ ID NOs 1-9) and/or the marker placental growths factor (PlGF) (SEQ ID NOs 10-12) is measured in addition to the markers of table 1 and possibly additional markers, as described herein. This further increases the accuracy of the method of the present invention.

The level of these markers sFlt-1 und PlGF may be determined using mass spectrometry or a quantitative immune assay, as known in the art.

In certain embodiments of the method, the measuring of the metabolite level (and possibly other markers) is performed using a mass spectrometer. In particular, a mass spectrometer may be used in combination with reversed phase high-performance liquid chromatography (HPLC).

In particular embodiments, a triple quadrupole mass spectrometer (TQMS) may be used in the method of the invention, i.e. a tandem mass spectrometer comprising two quadrupole mass analyzers in series, with a radio frequency-only quadrupole between them to act as a cell for collision-induced dissociation, as known in the art.

The present invention provides an in-vitro diagnostic test system (IVD) that is trained, as described further below, for the prediction of preeclampsia. For example, in order to determine whether a subject has or will develop preeclampsia, reference metabolite level values for preeclampsia positive and negative samples are determined. The metabolites can be quantitatively measured on an adequate set of training samples comprising cases and controls, and with adequate clinical information on preeclampsia status, applying adequate quality control measures, and on an adequate set of test samples, for which the detection is yet to be made. With such quantitative values for the metabolites and the clinical data for the training samples, a classifier can be trained and applied to the test samples to calculate the probability of the future presence or non-presence of preeclampsia during pregnancy.

Various classification schemes are known for classifying samples between two or more classes or groups, and these include, without limitation: Naive Bayes, Support Vector Machines, Nearest Neighbors, Decision Trees, Logistics, Artificial Neural Networks, and Rule-based schemes. In addition, the predictions from multiple models can be combined to generate an overall prediction. Thus, a classification algorithm or "class predictor" may be constructed to classify samples. The process for preparing a suitable class predictor is reviewed in R. Simon, Diagnostic and prognostic prediction using gene expression profiles in high-dimensional microarray data, British Journal of Cancer (2003) 89, 1599-1604.

In this context, the invention teaches an in-vitro diagnostic test system (IVD) that is trained in the prediction of a preeclampsia referred to above, comprising at least 5 metabolites, which can be quantitatively measured on an adequate set of training samples comprising cases and controls, with adequate clinical information on preeclampsia status, applying adequate quality control measures, and on an adequate set of test samples, for which the detection yet has to be made. Given the quantitative values for the metabolites and the clinical data for the training samples, a classifier can be trained and applied to the test samples to calculate the probability of the presence or absence of preeclampsia.

In the method of the invention, the level of several metabolite molecules is determined in a relative or an absolute manner, wherein an absolute measurement of metabolite level is preferred. The level is, if applicable, compared with that of other individuals, or with multivariate quantitative thresholds.

The conclusion whether the subject will develop preeclampsia or not is preferably reached on the basis of a classification algorithm, which can be developed using, for example, a random forest method, a support vector machine (SVM), or a K-nearest neighbor method (K-NN), such as a 3-nearest neighbor method (3-NN), as known in the art.

In certain embodiments of the method of the invention, the decision whether the subject has preeclampsia comprises the step of training a classification algorithm on a training set of cases and controls, and applying it to measured metabolite levels.

The determination of the level of the metabolites described herein is performed from blood samples using quantitative methods. For example, metabolite levels can be measured by mass spectrometry.

The blood sample used in the method of the invention may be, for example, a whole blood sample, a serum sample or a plasma sample. Metabolites are usually isolated by solid-phase column extraction from a certain volume of whole blood, serum, or plasma. In certain embodiments, serum or plasma samples are used. In certain embodiments, the metabolites are isolated by solid-phase column extraction plasma or serum.

In certain embodiments of the method of the invention, the method uses 5 or more markers, in particular 5, 6, 7, 8, 9, 10, 11, 12, 13, 14, 15, 16, 17, 18, 19, 20, 21 or more markers. In certain embodiments of the method, at least 5, at least 6, at least 7, at least 8, at least 9, at least 10, at least 11, at least 12, at least 13, at least 14, at least 15, at least 20, at least 25 or at least 30 markers are used.

When it is concluded in the method based on the measured levels of metabolites whether the subject is likely to develop preeclampsia, the subject may be treated by a physician. One option of treatment may be the administration of acetylsalicylic acid (ASA, Aspirin^{®}) to the subject in an appropriate dose. For example, 100 mg ASA per day may be administered. Administration may be started before gestational week 16, but could also be administered later or earlier.

In another aspect, the invention refers to the use of a method as described herein for the prediction of preeclampsia in a pregnant female human subject based on metabolites from a blood sample, in particular from a plasma sample or serum sample.

The invention also refers to the use of a marker for determining the risk of preeclampsia in a pregnant woman in the first trimester of pregnancy, comprising measuring the concentration of at least the markers listed in table 1. Such a method is performed in vitro.

In certain aspects, the invention is directed to a kit for detecting the level of metabolites in the subject's blood sample, where this "profile" allows for the conclusion of whether the subject will develop preeclampsia or not. In another aspect, the invention relates to a standard that allows for the detection of the metabolites associated with preeclampsia, in particular those described herein.

In another aspect, the invention refers to a kit for the prediction of preeclampsia in a female human subject based on metabolites obtained from a blood sample, comprising means for measuring the level of at least the markers listed in table 1.

The kit may comprise or contain at least one compound of table 1 as a standard for use in mass spectrometry. The standard can be used to ensure quantitative reproducibility of the method of the invention.

In certain embodiments, the kit comprises or contains 1, 2, 3, 4, or 5 compounds of table 1 as a standard for use in mass spectrometry.

The kit may also comprise at least one compound of table 2 and/or table 3 as a standard for use in mass spectrometry.

The solution of the standard compound of table 1, 2 and/or 3 is usually prepared gravimetrically and is supplied in a deactivated glass ampule sealed under an inert gas, such as argon. The concentration is usually verified by comparison to an independently prepared calibration standard.

In another aspect, the invention refers to the use of such a kit for the prediction of preeclampsia in a human subject based on metabolites from a blood sample, comprising means for measuring the level of at least the markers listed in table 1. The kit may be used in a method for predicting preeclampsia as described herein.

In certain embodiments, the standard is a marker as listed in table 1, table 2 and/or table 3 that is isotope labeled to make it distinguishable from the marker of the sample that is to be measured, such that the standard can be measured simultaneously to the sample in a mass spectrometer.

In certain embodiments, the standard is deuterated, it contains one or more, in particular one deuterium atom instead of a hydrogen atom.

In another aspect, the invention refers to the use of a mass spectrometer for performing a method for the prediction of preeclampsia as described herein.

In another aspect, the invention refers to a method for preparing a metabolite profile that is indicative of the presence or absence of preeclampsia in a subject, comprising:
- isolating metabolites from a blood sample obtained from the subject, and
- measuring the level of the following metabolites in the sample: 14,15-DHET, 13-HODE, 10,11-EDP, 21-HDHA, and 11,12-DHET.

In yet another aspect, the invention refers the use of a set of markers for determining within the first trimester of pregnancy the risk for a pregnant woman to suffer from preeclampsia, comprising measuring the level of the following metabolites in the sample: 14,15-DHET, 13-HODE, 10,11-EDP, 21-HDHA, and 11,12-DHET.

### Examples

The inventors measured eighty-one free circulating eicosanoids derived from linoleic-, eicosapentaenoic-, docosahexaenoic- and arachidonic acids by Triple-Quad-Tandem mass spectrometry in serum collected at gestational week 12 in a cohort study (100 total, 50 cases, 50 controls). The preeclampsia phenotype was heterogeneous with predominantly mild, late-onset disease. Using a regression-tree approach, prediction models incorporated clinical data, angiogenic markers and eicosanoid metabolites, compared with a model including only standard clinical variables in combination with sFlt-1 and PlGF.

Here, the term "eicosanoids" is used to include oxylipins that are derived from arachidonic acids (AA), linoleic acid (LA), or eicosapentaenoic- and docosahexaenoic-acid (EPA/DHA). Metabolites are generated following phospholipase A2 (PLA2)-mediated release by cyclooxygenases (COX), lipoxygenases (LOX), cytochrome P450 (CYP) enzymes, or autoxidation. Dietary enrichment with EPA/DHA from fish and fish oil derivatives is known to promote cardio-protective effects.

A regression tree approach for predicting preeclampsia in the first trimester demonstrated good predictive value when combining clinical data, maternal blood pressure and sFlt-1/PlGF; sensitivity 56, specificity 94, AUC 78. However, the addition of eicosanoid metabolites substantially increased the overall performance of a regression-tree approach, sensitivity 71, specificity 99, AUC 87.

### Materials and Methods

### Subjects and characterization

The case-control study was nested in the prospective, population-based Odense Child Cohort, a cohort including newly pregnant women between January 1^{st} 2010 and December 31^{st} 2012 in Odense, Denmark. All participants gave written informed consent. The study complied with the Helsinki declaration and was approved by the Regional Scientific Ethical Committee for Southern Denmark (no. S-20090130).

Longitudinal serum samples from early and late pregnancy (median 12 weeks of gestation and 29 weeks of gestation) were included from 52 preeclamptic women and 51 healthy pregnancies, matched on gestational age at blood sampling, body mass index, parity, twinning pregnancies, and smoking status. The criteria for preeclampsia were repeated measures of hypertension (>140/90 mmHg) after 20 weeks of gestation with at least +1 for proteinuria on sterile urine dipstick. The more severe subtypes were further defined; early-onset PE (EOPE) with debut before 34+0 weeks of gestation, and preeclampsia with preterm delivery (PreEOPE) before 37+0 weeks of gestation.

### Eicosanoid measurement

This HPLC/MS/MS based method (Lipidomix, Germany) comprised all COX, LOX, CYP and auto-oxidation generated free eicosanoids originated from the substrates AA, LA or EPA/DHA. Eicosanoids were isolated by Varian Bond Elute Certify II solid-phase column extraction from 350 µl serum. Extracts were analyzed by Triple-Quad-Tandem mass spectrometry Agilent 6460 (Agilent Technologies, Santa Clara, CA) coupled with a rapid resolution HPLC Agilent 1200.

### Angiogenic factor measurements

Measurements for BRAHMS sFLT-1 and PIGF KRYPTOR immune assays were performed on the fully automated KRYPTOR compact Plus system (KRYPTOR PIGF and KRYPTOR sFLT-1; Thermo Fisher Scientific) as described in Andersen et al., 2016.

### Statistical methods

All quantitative variables were tested for Gaussian distribution. Descriptive statistics are mean and SD or median and 25^{th}/75^{th} percentile as appropriate. Group differences were tested by t-test or Wilcoxon-test. For time differences, pairwise tests were applied. Correlations between clinical parameters and metabolites were analyzed by Spearman rank correlations and plotted in correlation heat maps. To determine diagnostic values, we applied receiver operating characteristic (ROC)-curves and logistic regressions. As base model, sFLT-1 and PIGF were included. This model was then extended by each metabolite separately. Metabolites significantly improving model fit were then entered into the final multivariate model. Areas under the curve (AUC) of base vs. final model were compared by one-side significance test. Prediction of disease groups by logistic regression makes the assumption of a linear risk increase per unit of predictor and cannot determine threshold effects or U-shaped risks. Therefore, regression trees as a non-linear threshold based classification approach were included. For this approach, no pre-selection of metabolites was applied. All analyses were conducted in R version 3.3.1 (R Core Team 2016: A language and environment for statistical computing R Foundation for Statistical Computing, Vienna, Austria; https://www.R-proj ect. org/).

### Figures

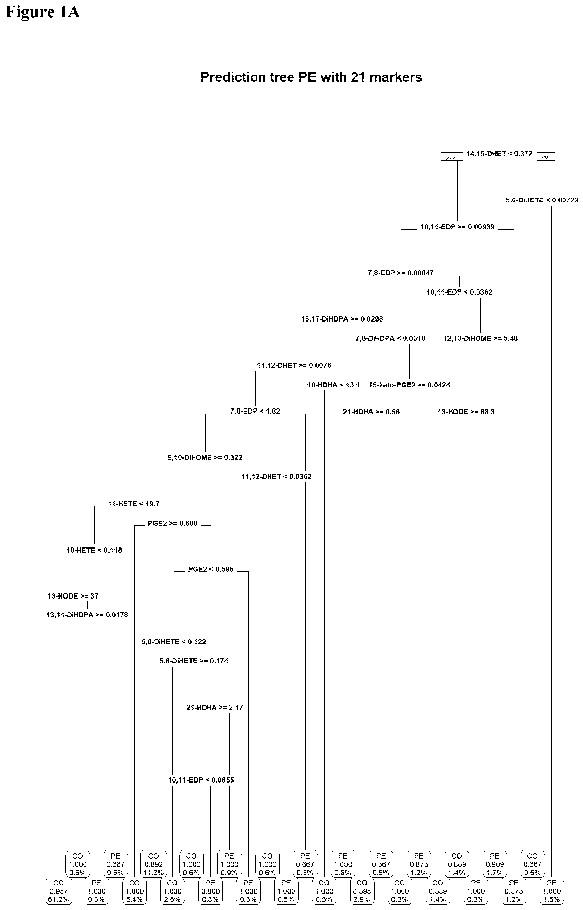

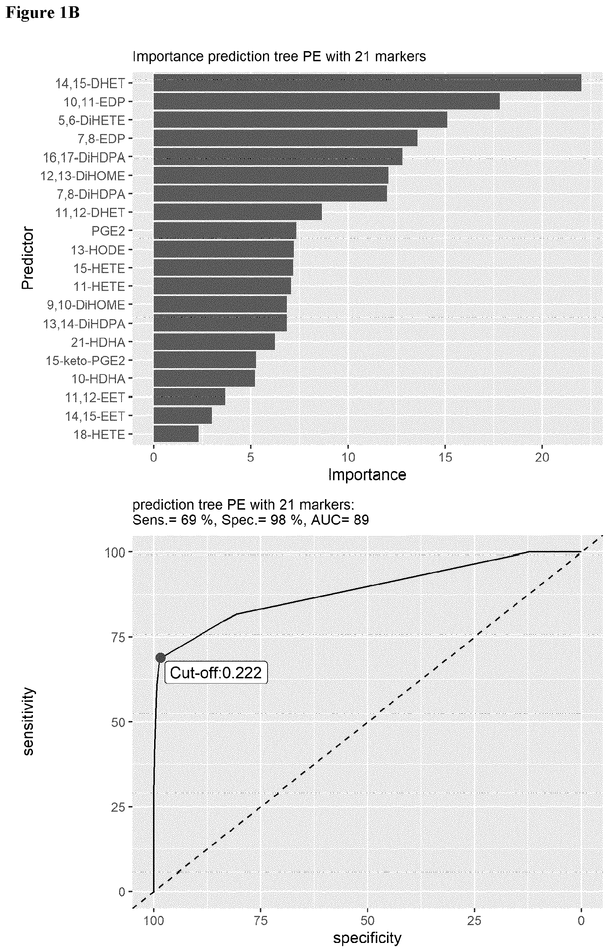

- **Figure 1**: **A** Decision tree of 21 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

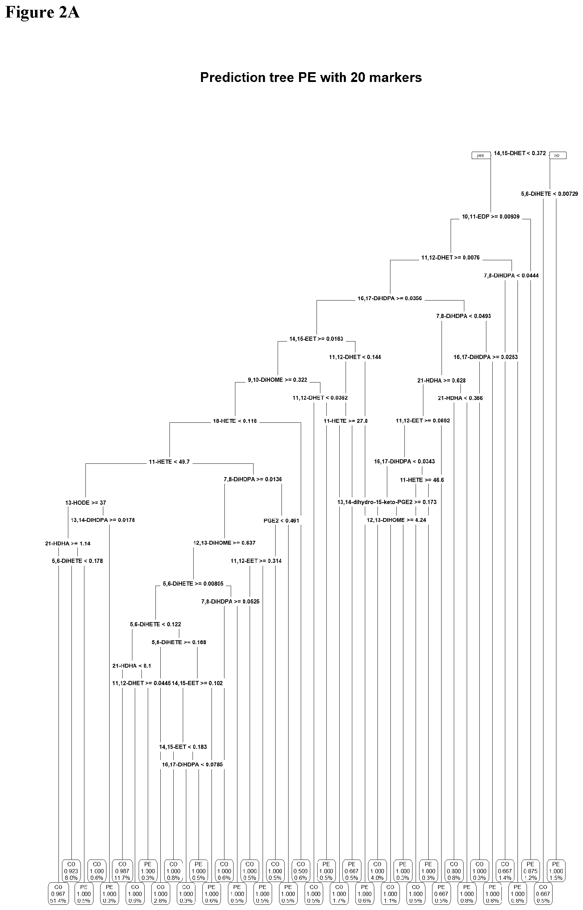

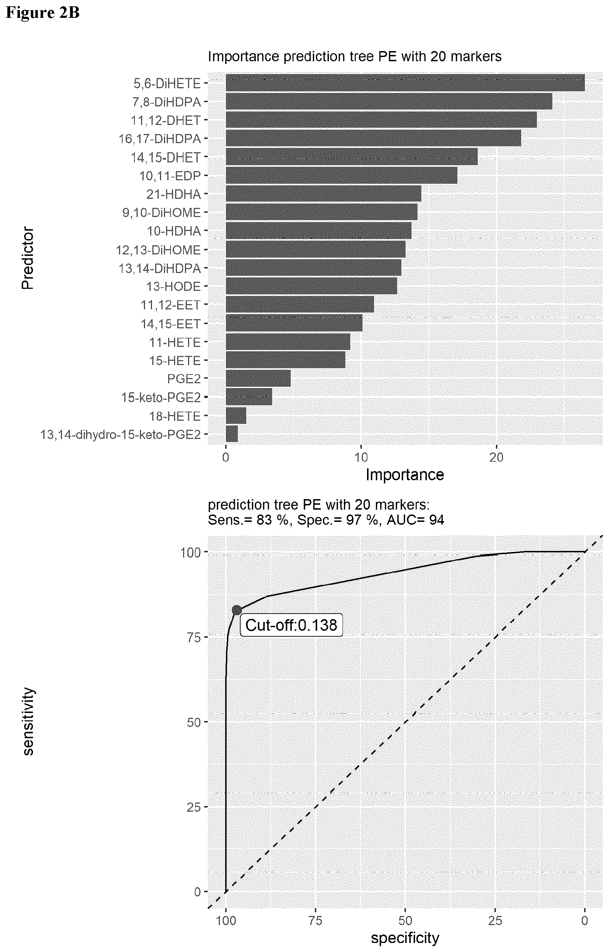

- **Figure 2**: **A** Decision tree of 20 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

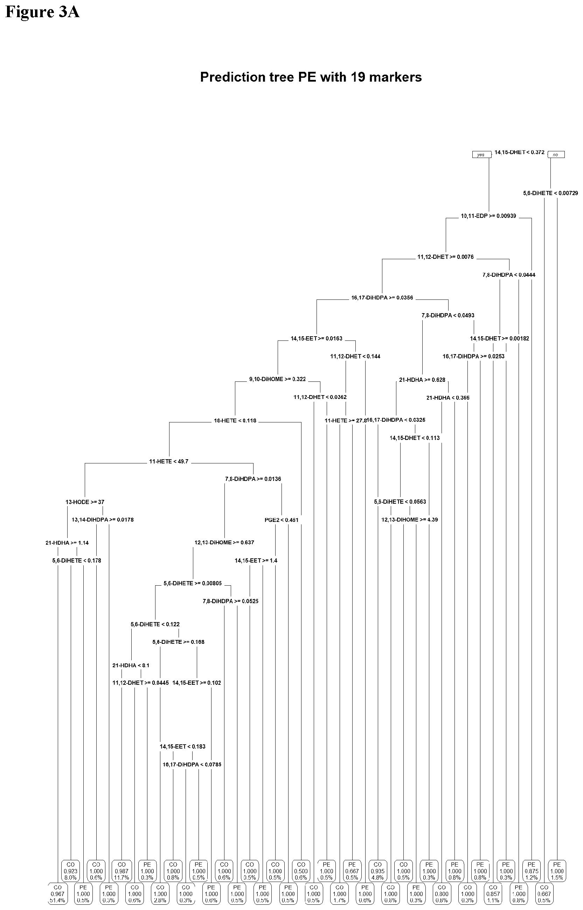

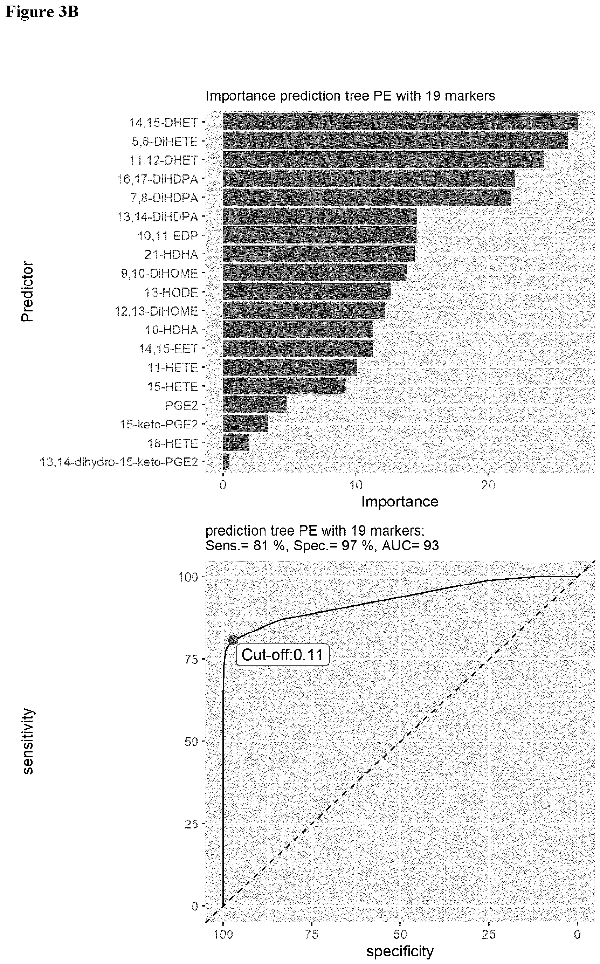

- **Figure 3**: **A** Decision tree of 19 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

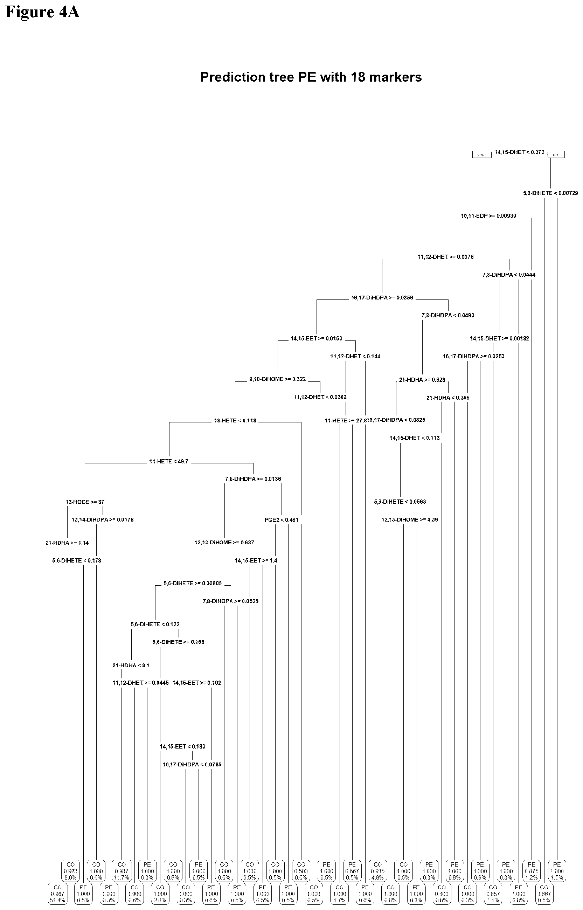

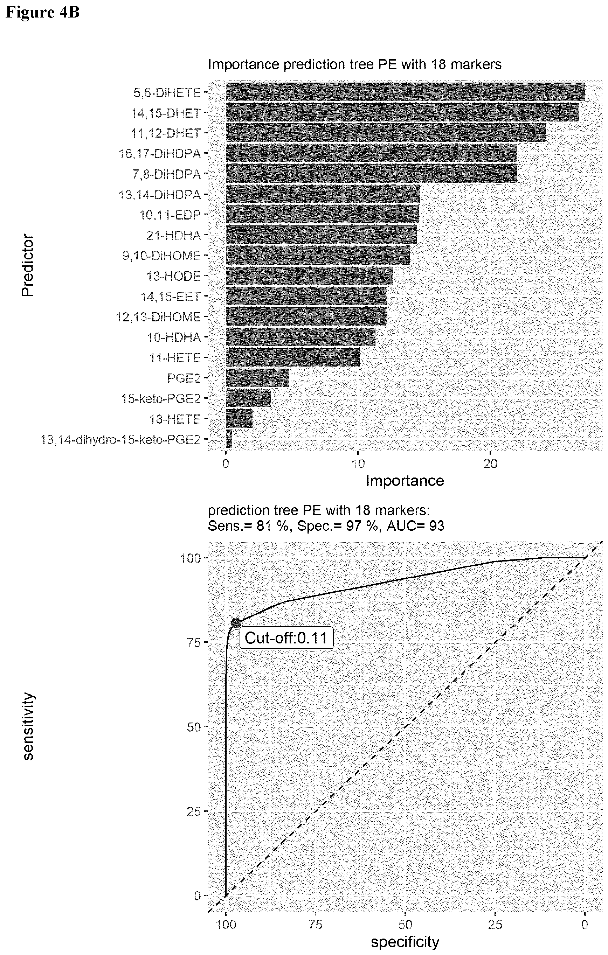

- **Figure 4**: **A** Decision tree of 18 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

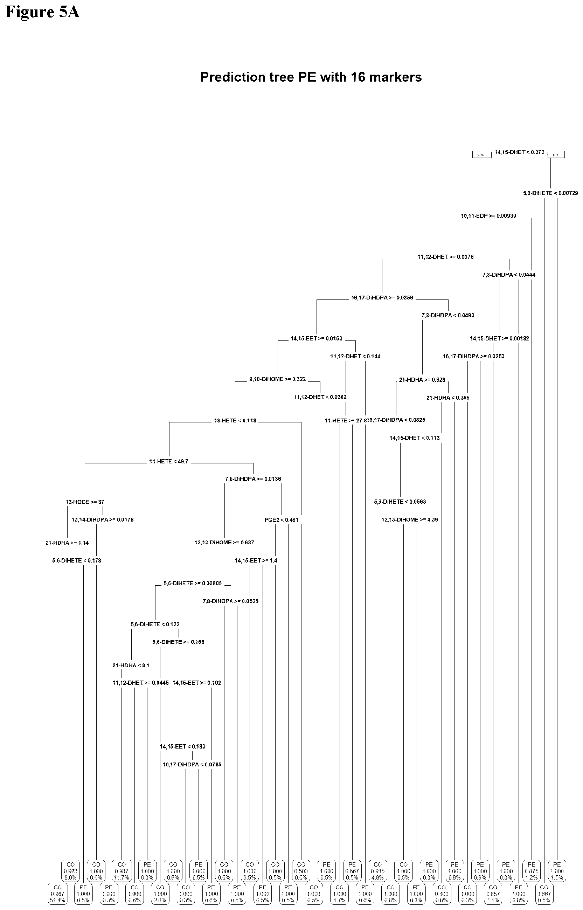

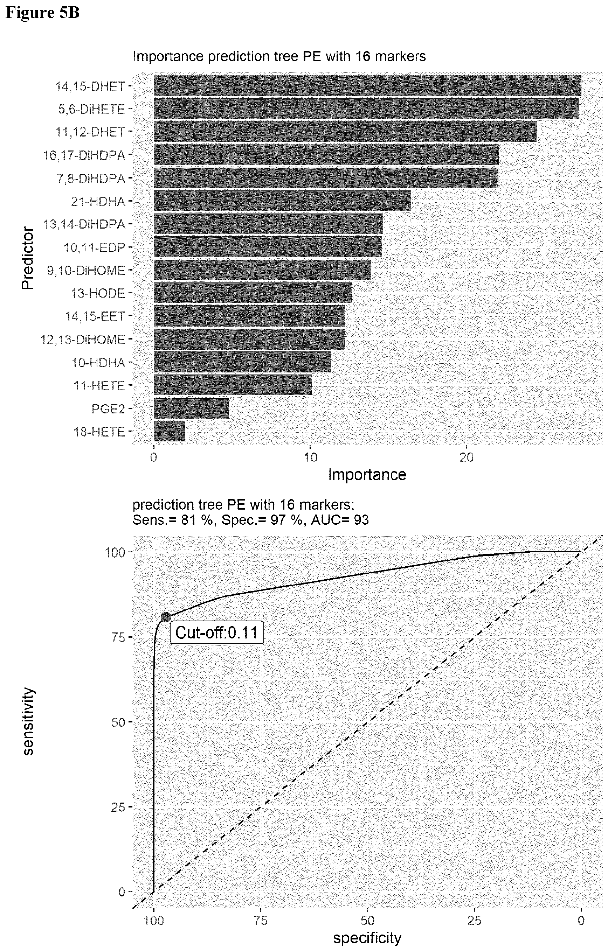

- **Figure 5**: **A** Decision tree of 16 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

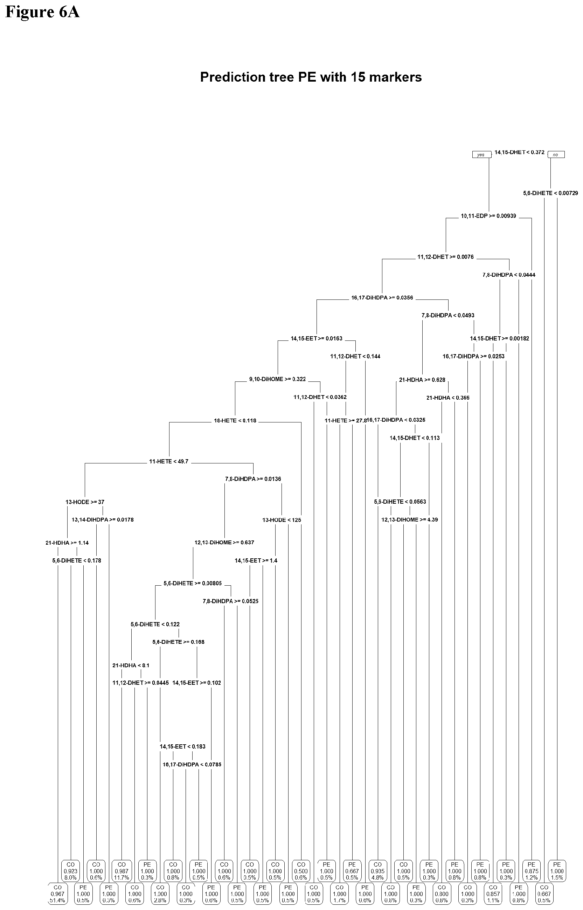

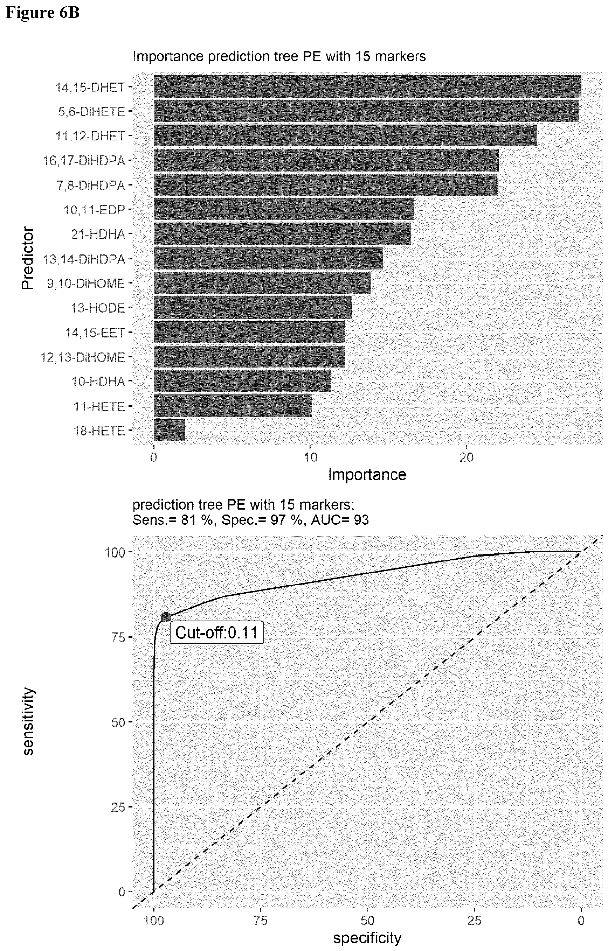

- **Figure 6**: **A** Decision tree of 15 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

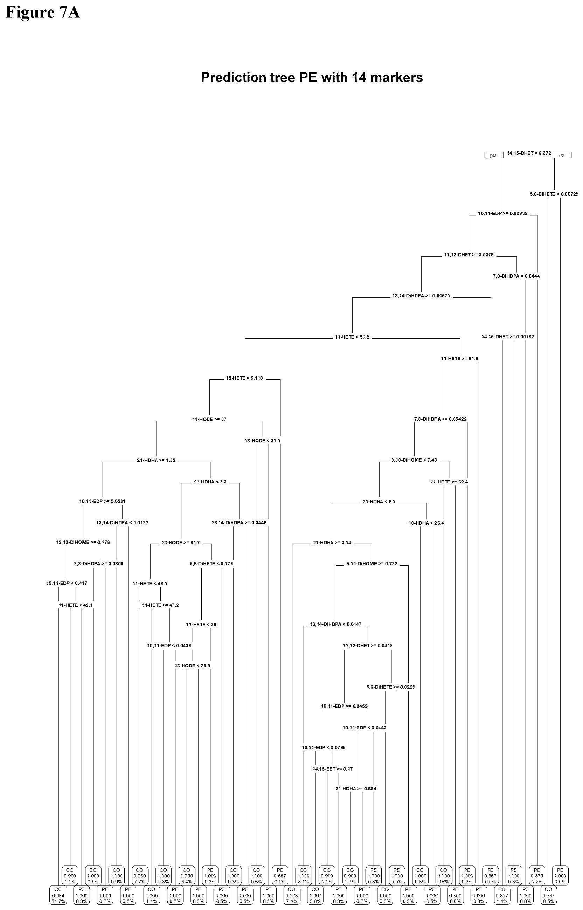

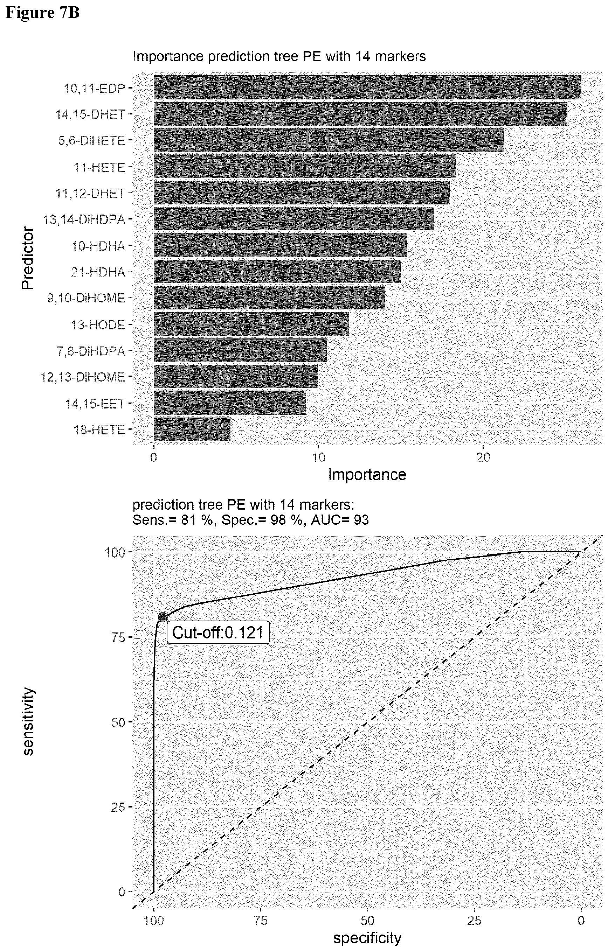

- **Figure 7**: **A** Decision tree of 14 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

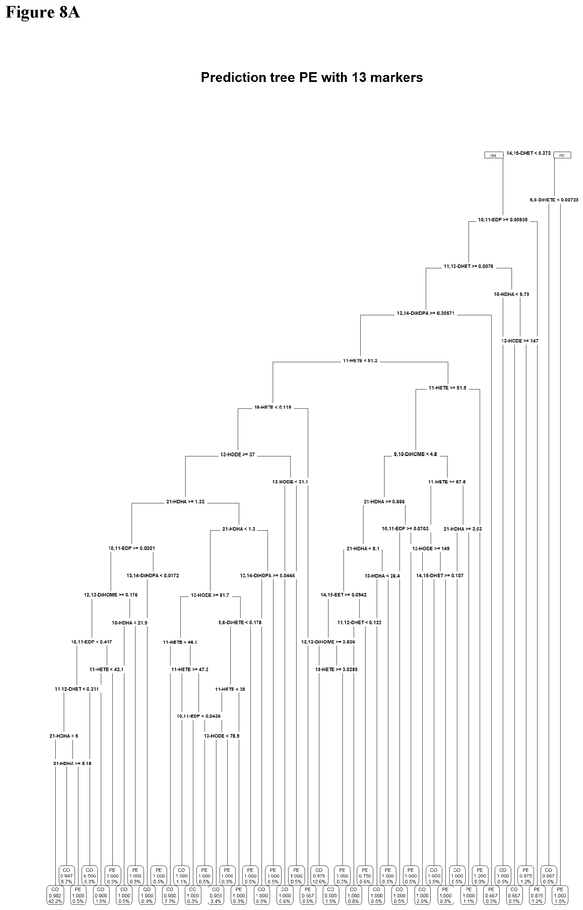

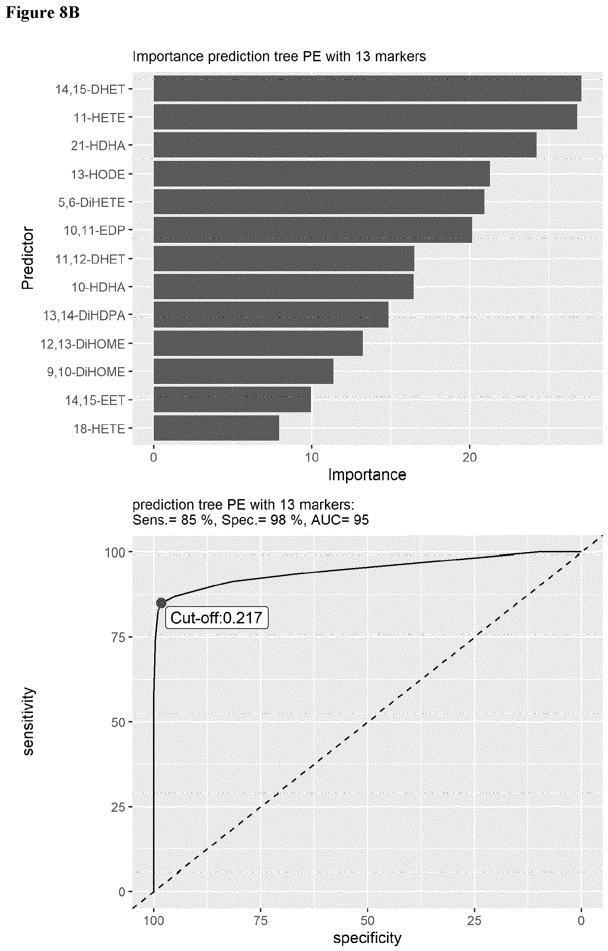

- **Figure 8**: **A** Decision tree of 13 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

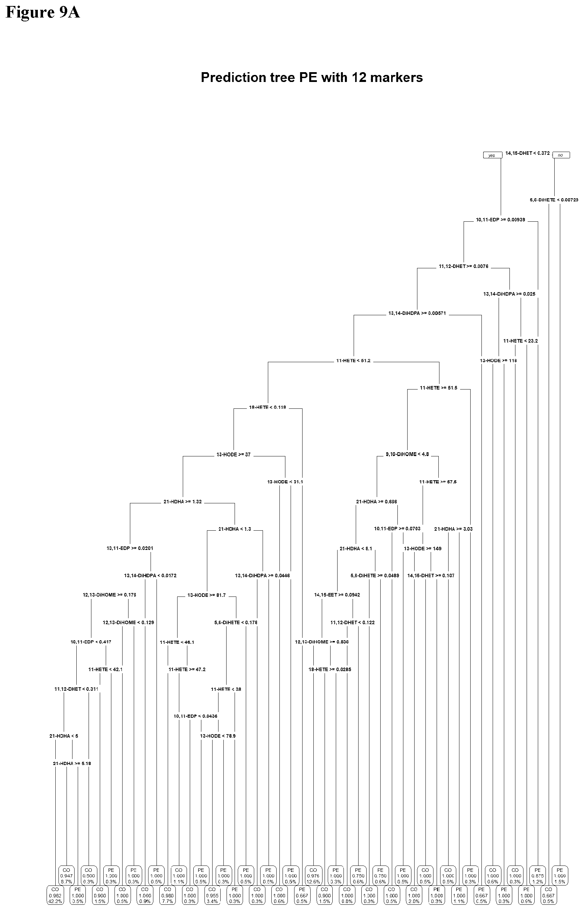

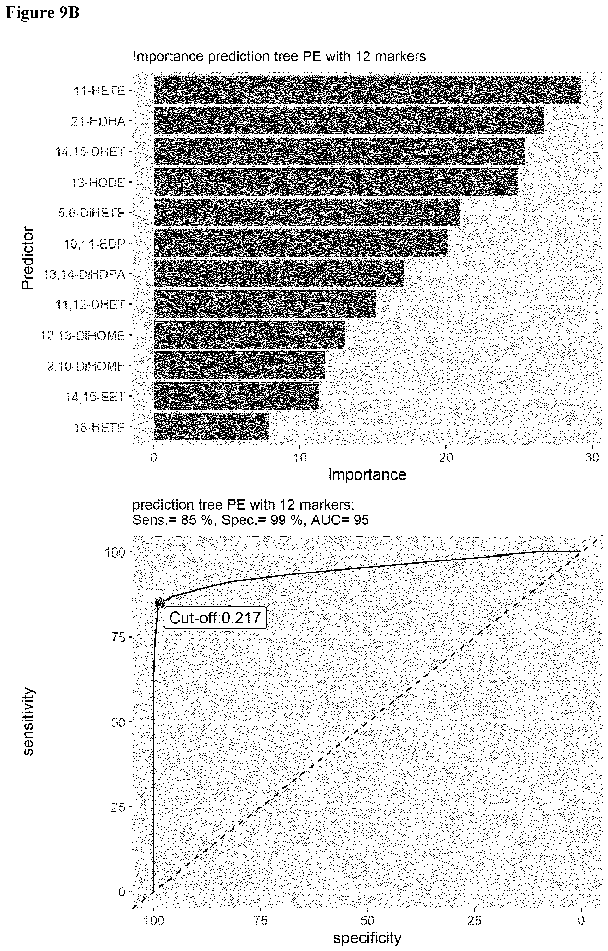

- **Figure 9**: **A** Decision tree of 12 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

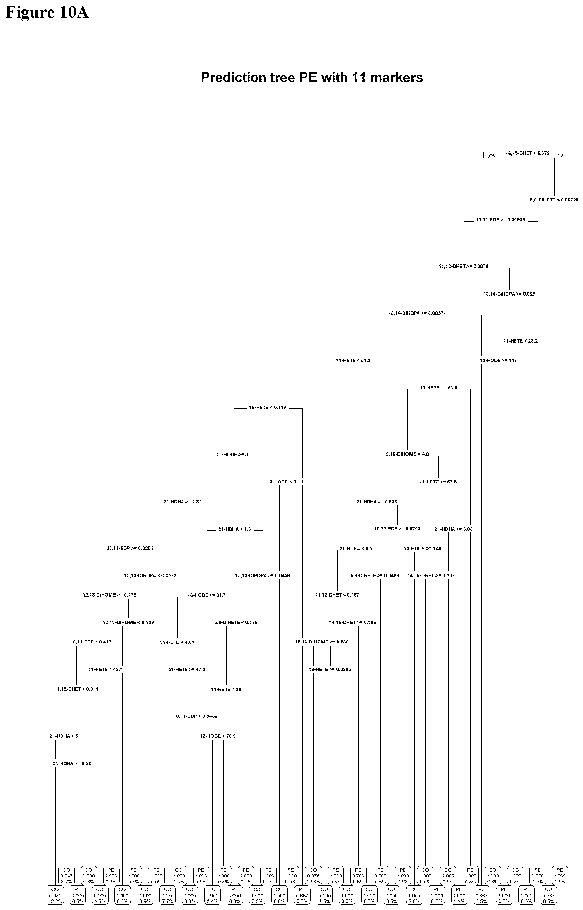

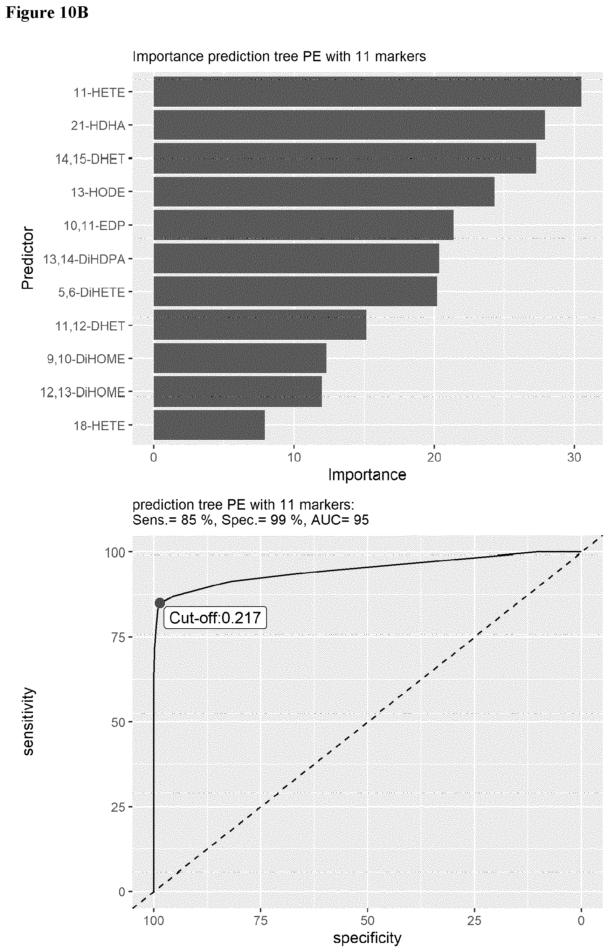

- **Figure 10**: **A** Decision tree of 11 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

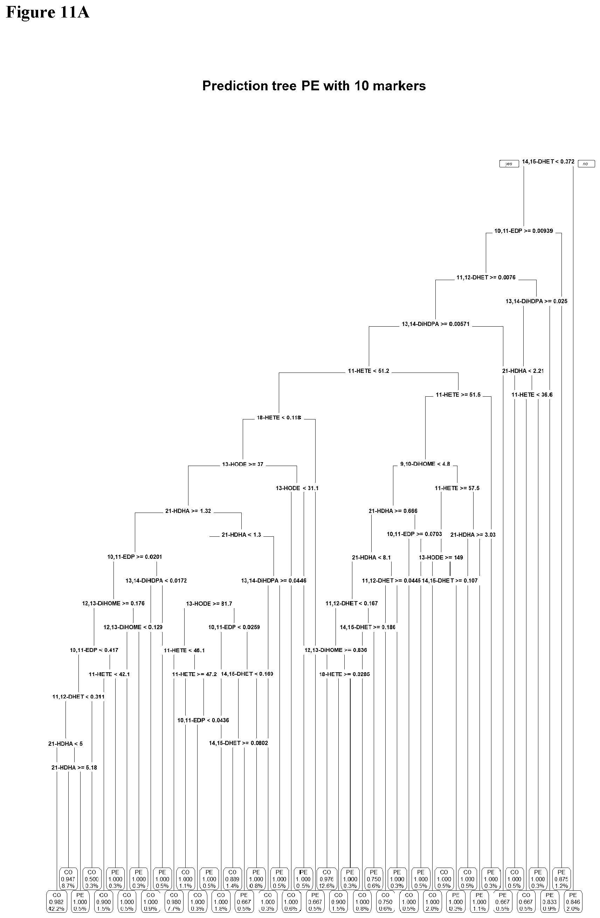

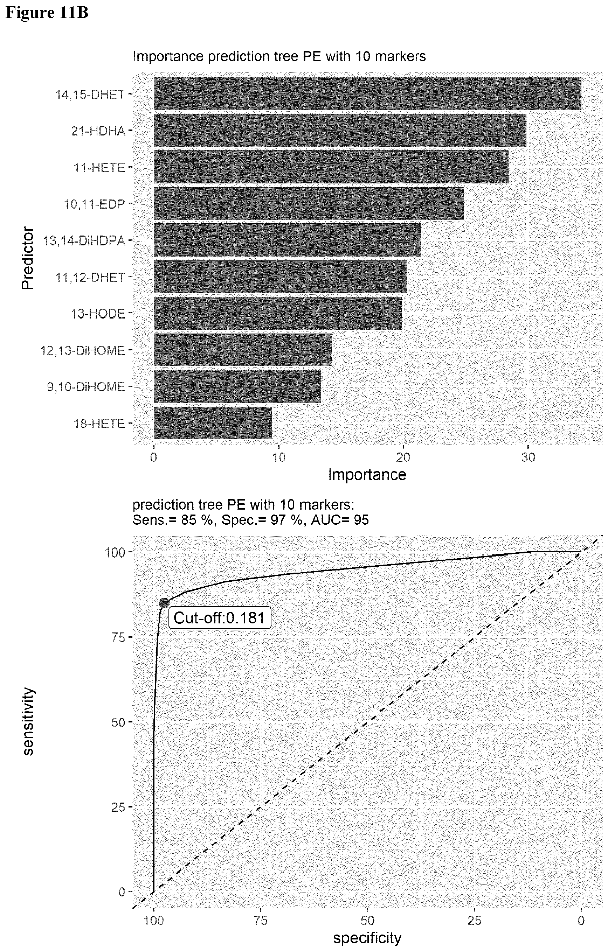

- **Figure 11**: **A** Decision tree of 10 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

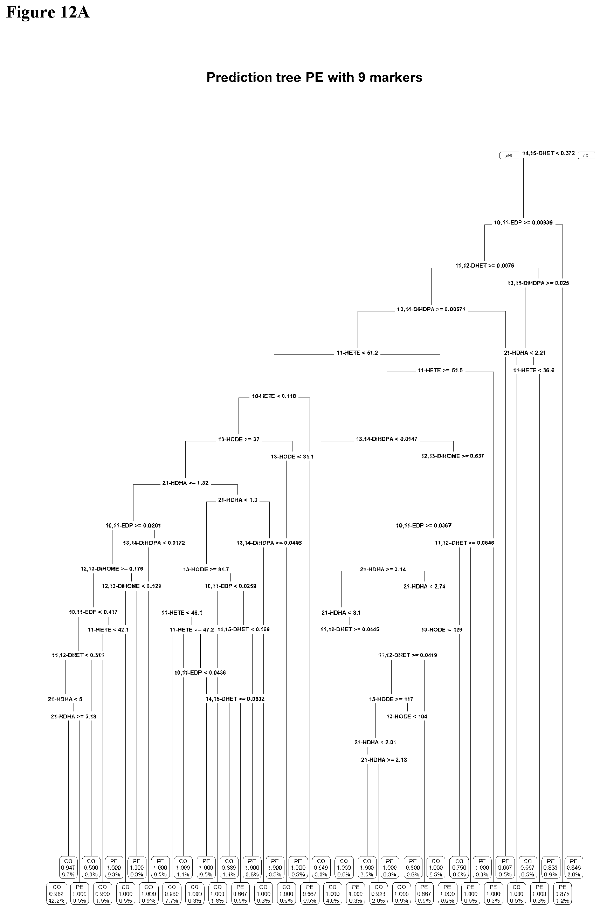

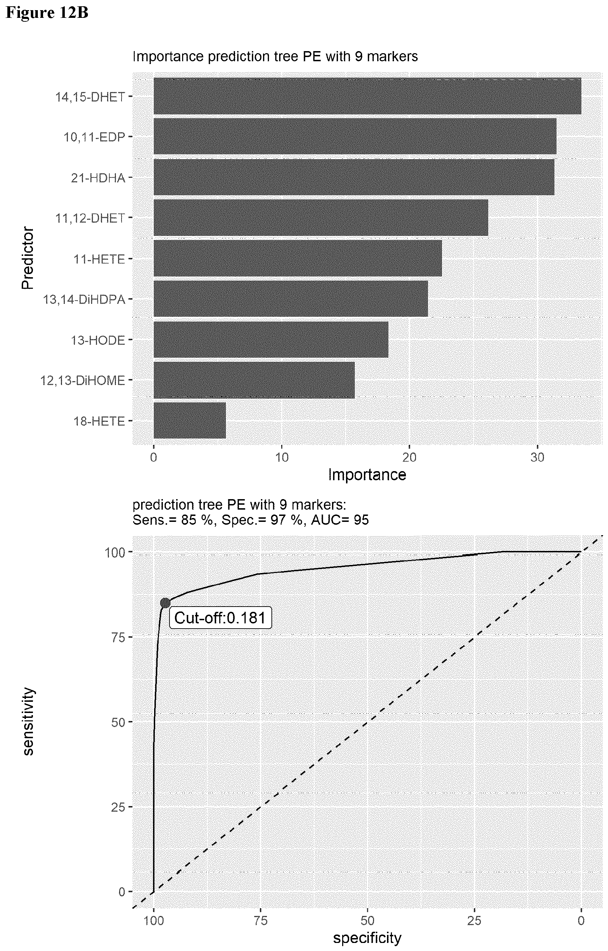

- **Figure 12**: **A** Decision tree of 9 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

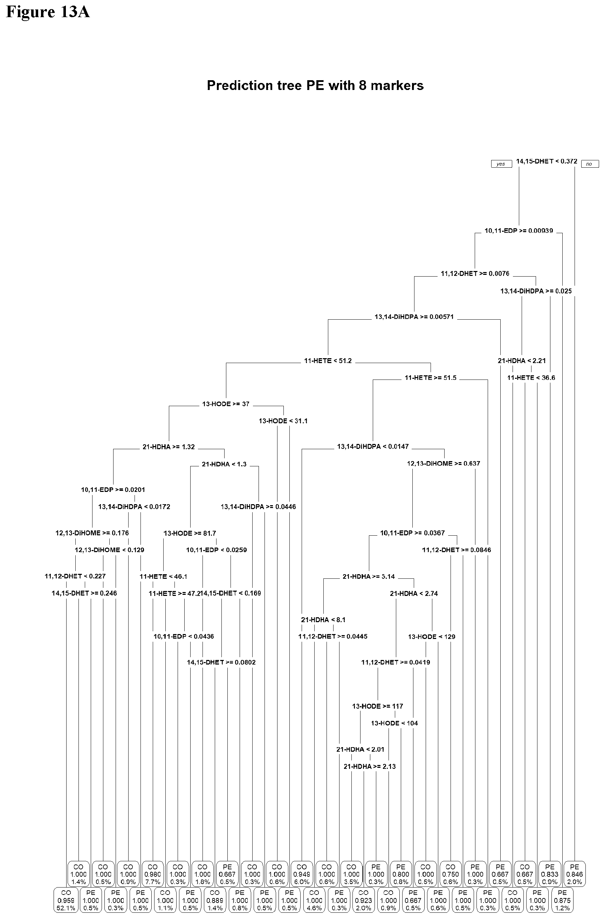

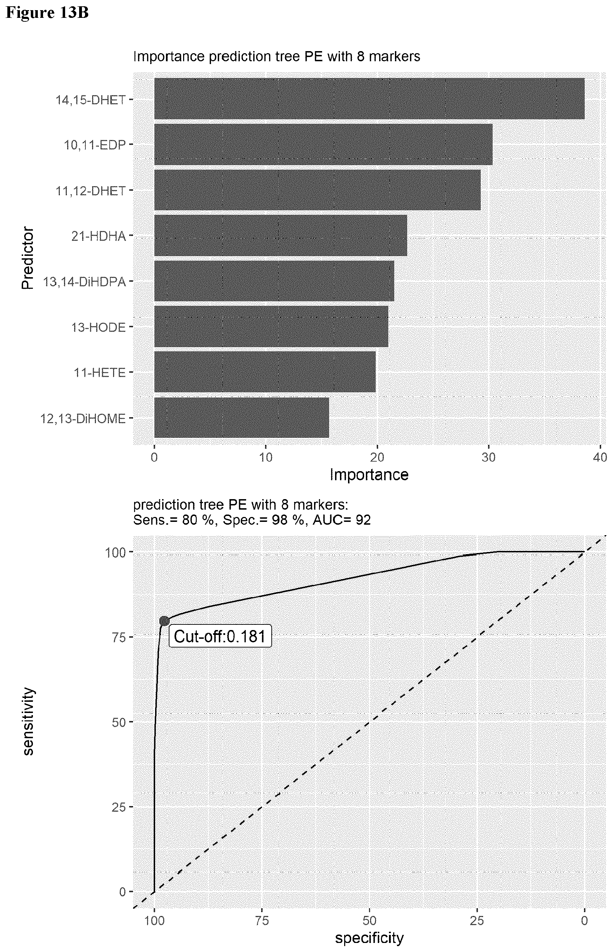

- **Figure 13**: **A** Decision tree of 8 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

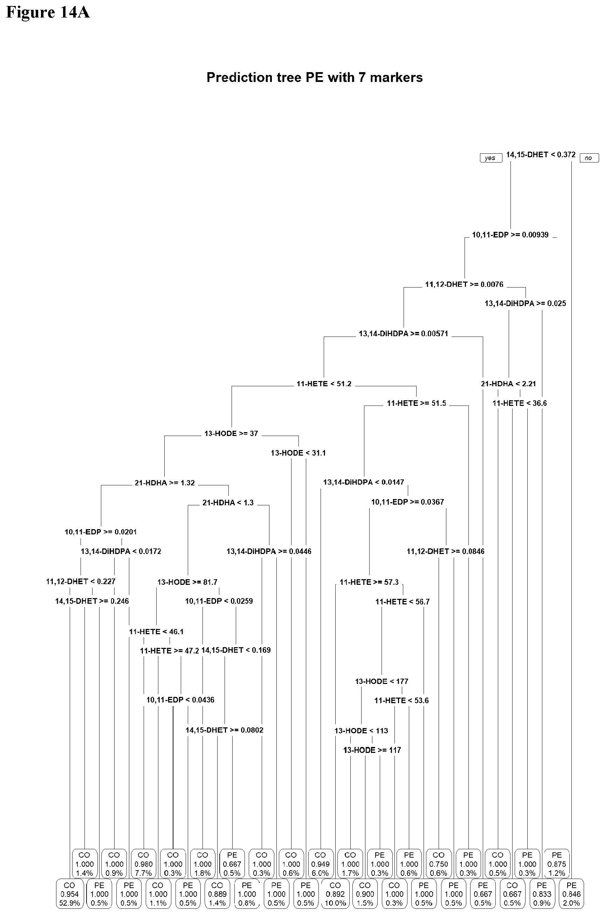

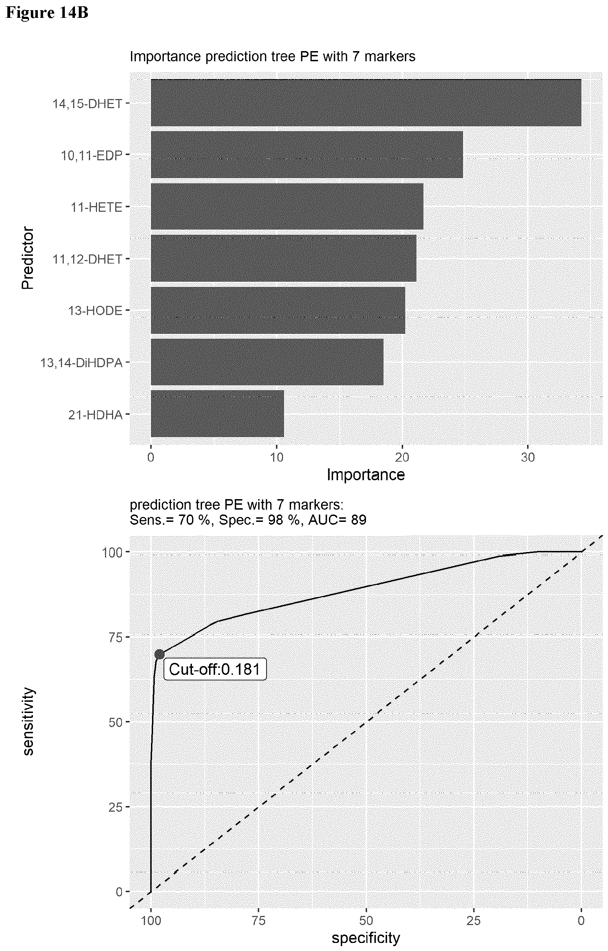

- **Figure 14**: **A** Decision tree of 7 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

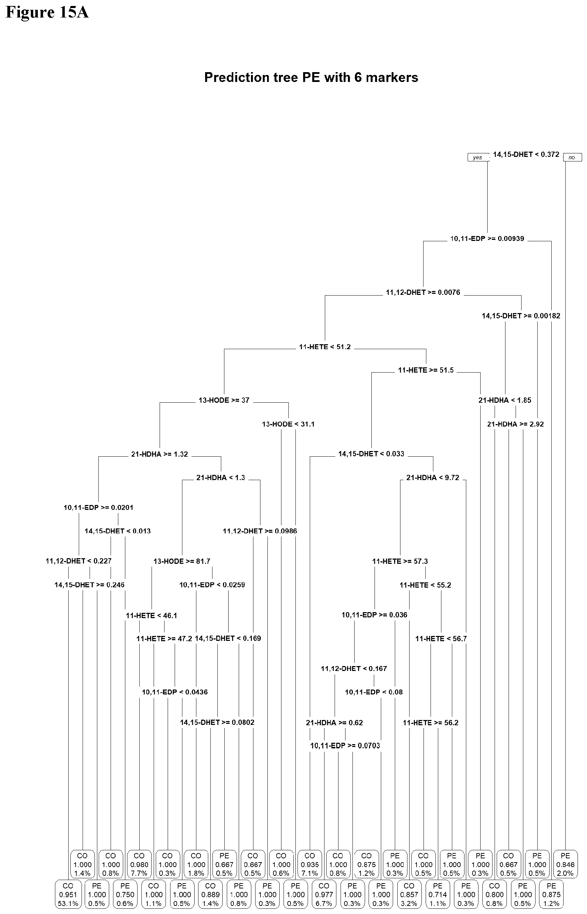

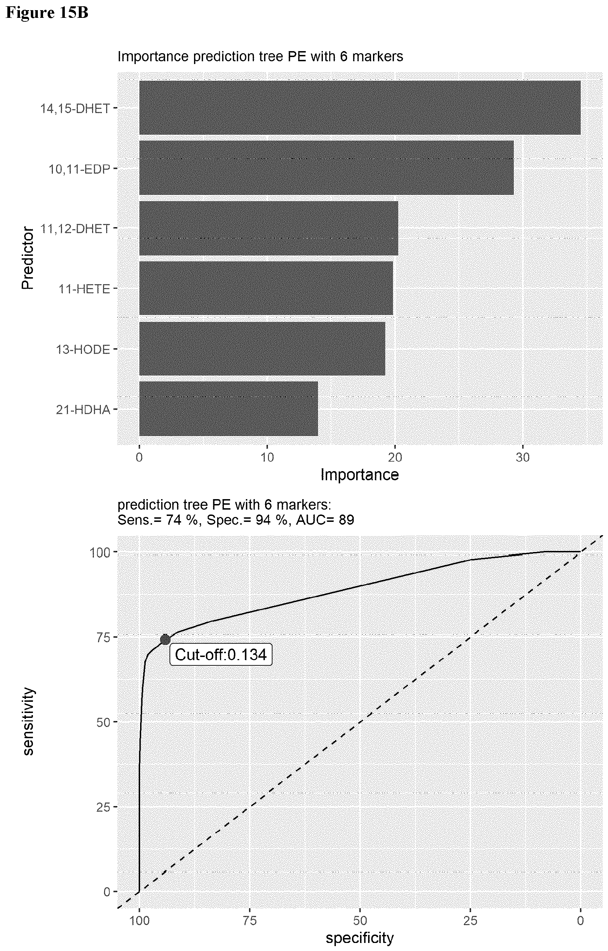

- **Figure 15**: **A** Decision tree of 6 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

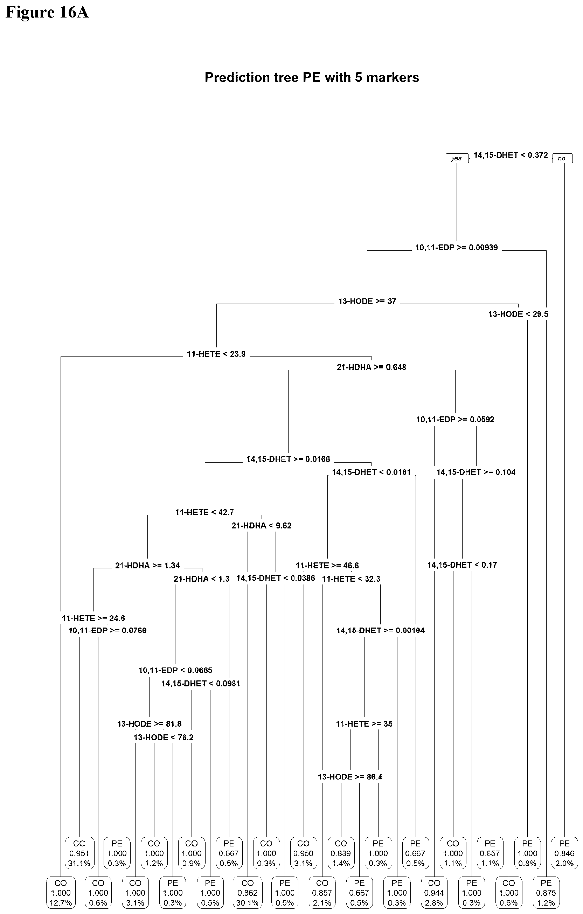

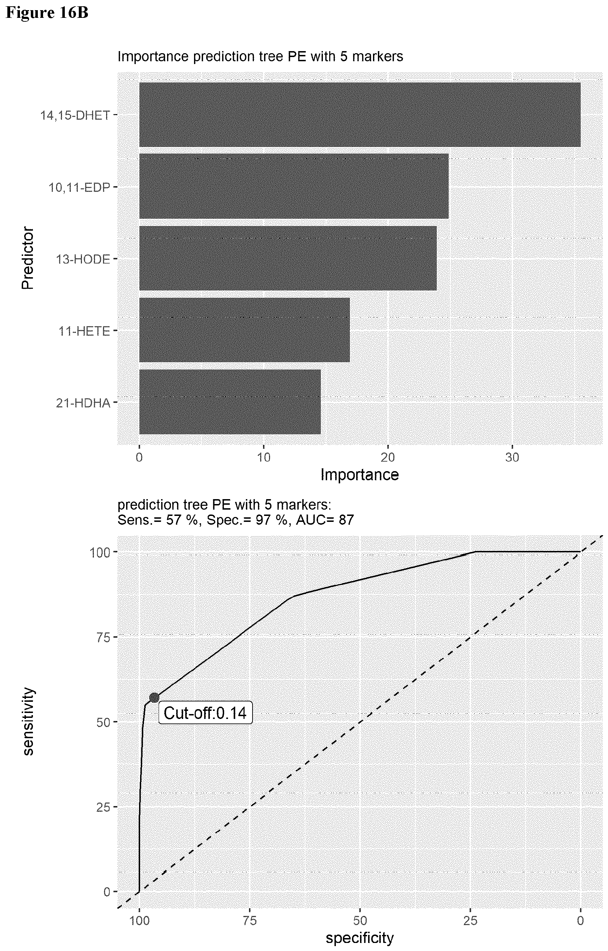

- **Figure 16**: **A** Decision tree of 5 markers with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

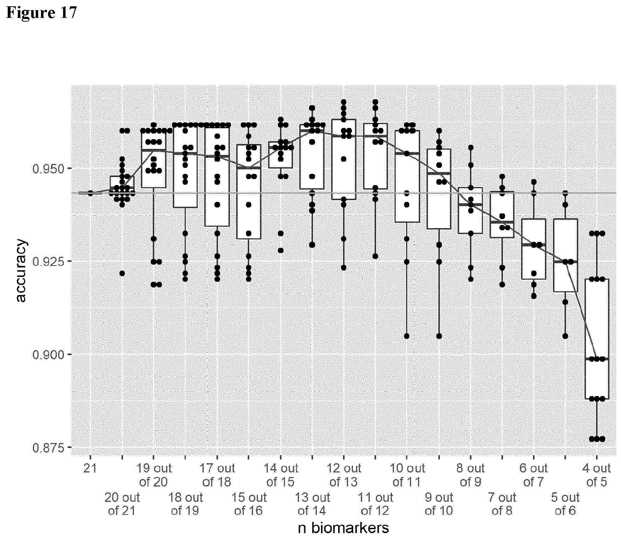

- **Figure 17**: Accuracy (% of correct classifications) for all possible combinations of subsamples of biomarkers. Red line shows median accuracy for a given number of biomarkers.

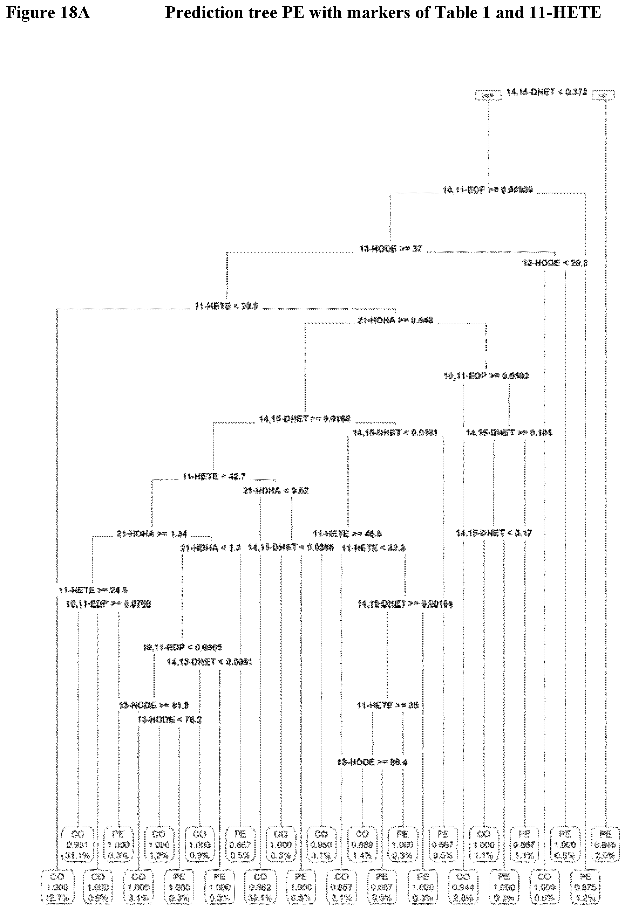

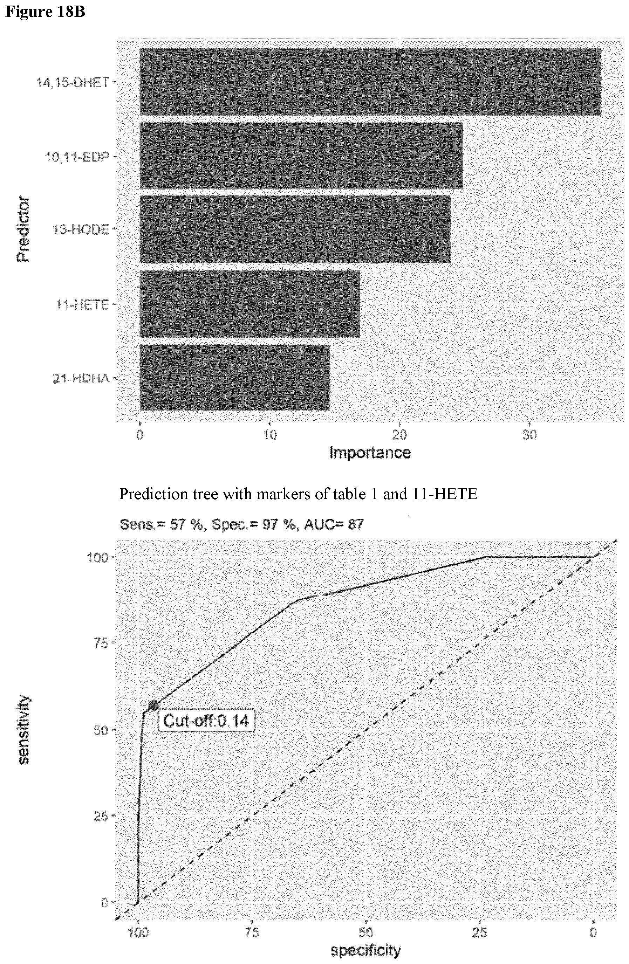

- **Figure 18**: Decision tree with markers of table 1 and 11-HETE with hierarchical definition of the selected marker and cut-of value for step-wise optimization of classification. Lower boxes represent the final classification of the respective branch of the decision tree with assigned diagnosis, relative frequency of correct classification, and % of total population assigned to that branch.
**B** (top): Importance of biomarkers for the final classification; as each marker can be used more than once in the decision tree, this is not identical to the tree hierarchy. (bottom): ROC-plot for sensitivity and specificity for the continuous outcome probabilities given by the decision tree. The optimal cut-off is selected to optimize both by computing the maximum distance from the diagonal.

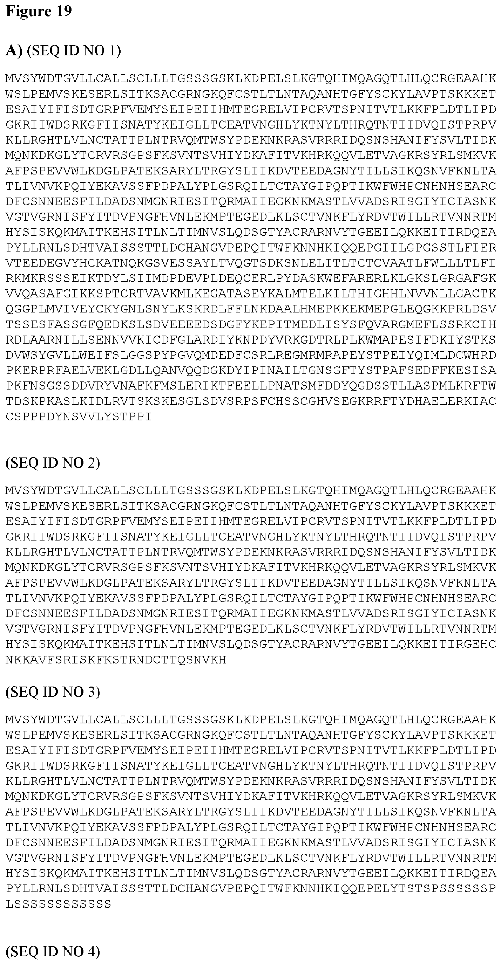

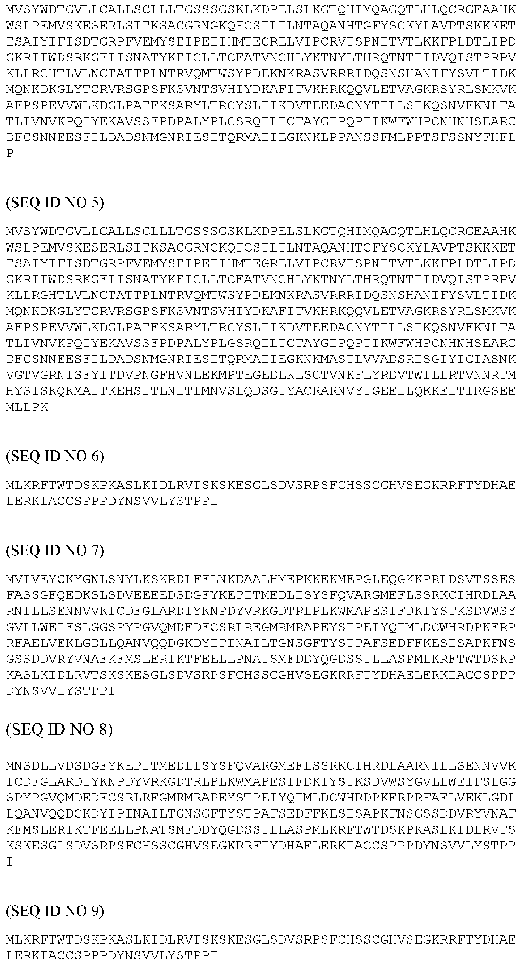

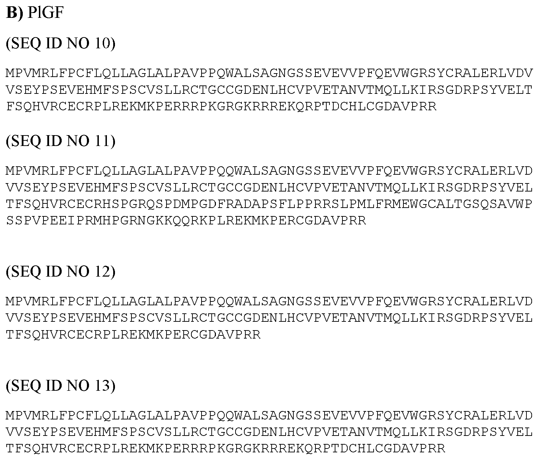

- **Figure 19**: **A** Human protein sequences of sFlt-1 (SEQ ID NO 1-9). Of this gene, 9 transcripts (splice variants), 198 orthologues, and 54 paralogues are known.
**B** Human protein sequences of PlGF (SEQ ID NO 10-13). Of this gene, 8 transcripts (splice variants), 225 orthologues and 4 paralogues are known.

## Claims

1. A method for the prediction of preeclampsia in a pregnant female human subject until the end of gestational week 16 of pregnancy based on metabolites from a blood sample obtained from said subject, comprising:
- measuring the level of the following metabolites in the sample: 14,15-DHET, 13-HODE, 10,11-EDP, 21-HDHA, and 11,12-DHET, and
- concluding based on the measured level whether the subject is likely to develop preeclampsia.

2. The method of claim 1, further comprising:
- measuring the level of at least one, in particular of at least five of the following metabolites in the sample:
7,8-EDP, 16,17-DiHDPA, 12,13-DiHOME, 11,12-DHET, 9,10-DiHOME, 5,6-DiHETE; 10-HDHA, 11,12-EET, 15-HETE, 7,8-DiHDPA, PGE2, 14,15-EET, 18-HETE, EODAcis, EODAtrans, and 13,14-DiHDPA.

3. The method of claim 1 or 2, further comprising:
- measuring the level of 11-HETE.

4. The method of claims 1 to 3, wherein the level of the following metabolites in the sample is measured: 14,15-DHET, 13-HODE, 10,11-EDP, 21-HDHA, and 11-HETE;
5,6-DiHETE; 11,12-DHET; 13,14-DiHDPA; 12,13-DiHOME; 9,10-DiHOME; and 18-HETE.

5. The method of claims 1 to 3, wherein the level of the following metabolites in the sample is measured: 14,15-DHET, 13-HODE, 10,11-EDP, 21-HDHA, and 11-HETE;
5,6-DiHETE; 11,12-DHET; 13,14-DiHDPA; 12,13-DiHOME; 9,10-DiHOME; 18-HETE; and 14,15-EET.

6. The method of claims 1 to 5, wherein the level of sFLT-1 and/or PlGF is measured.

7. The method of claims 1 to 6, wherein the method is performed within the first trimester of pregnancy.

8. The method of claims 1 to 7, wherein the measuring of metabolite levels is performed using a mass spectrometer.

9. The method of claims 1 to 8, wherein the decision whether the subject has preeclampsia comprises the step of training a classification algorithm on a training set of cases and controls, and applying it to measured metabolite levels, in particular wherein the classification method is a random forest method, a support vector machine (SVM), or a K-nearest neighbor method (K-NN), such as a 3-nearest neighbor method (3-NN).

10. Use of a method of claims 1 to 7 for the prediction of preeclampsia in a pregnant female human subject based on metabolites from a blood sample, in particular from a plasma sample or serum sample.

11. A kit for the prediction of preeclampsia in a female human subject based on metabolites obtained from a blood sample, comprising samples of the following metabolites: 14,15-DHET, 13-HODE, 10,11-EDP, 21-HDHA, and 11,12-DHET, in particular for use as a standard in mass spectrometry.

12. The kit of claim 11, wherein the sample carries a marker that allows for the differentiation from the naturally occurring metabolite in mass spectrometry, in particular wherein the marker is deuterium.

13. Use of a kit of claim 11 or 12 for the prediction of preeclampsia in a human subject based on metabolites from a blood sample, in particular in a method of claims 1 to 9, comprising means for measuring the level of at least the following metabolites in the sample, comprising
- using samples of 14,15-DHET, 13-HODE, 10,11-EDP, 21-HDHA, and 11,12-DHET in order to measure the level of 14,15-DHET, 13-HODE, 10,11-EDP, 21-HDHA, and 11,12-DHET in the blood sample.

14. A method for preparing a metabolite profile that is indicative of the presence or absence of preeclampsia in a subject, comprising:
- isolating metabolites from a blood sample obtained from the subject, and
- measuring the level of the following metabolites in the sample: 14,15-DHET, 13-HODE, 10,11-EDP, 21-HDHA, and 11,12-DHET.

15. Use of a set of markers for determining the risk for a pregnant woman to suffer from preeclampsia, comprising
- measuring the level of the following metabolites in the sample: 14,15-DHET, 13-HODE, 10,11-EDP, 21-HDHA, and 11,12-DHET.
